# FASCICULE DE BREVET EUROPEEN

(11) **EP 2 384 329 B1**
(45) Date de publication et mention de la délivrance du brevet: **23.05.2018**
(21) Numéro de dépôt: 09806180.7
(22) Date de dépôt: 28.12.2009
(51) Int. Cl.: C07F 5/02, G01N 33/68, C07K 14/47, C07H 23/00, C07F 9/6596

(54) **PROCEDE DE PREPARATION DE COMPOSES DIPYRROMETHENES-BOROSUBSTITUES FLUORESCENTS ET LEUR UTILISATION POUR LE DIAGNOSTIC**
VERFAHREN ZUR HERSTELLUNG VON FLUORESZENTE, BORSUBSTITUIERTE DIPYRROMETHENE UND DEREN VERWENDUNG FÜR DIE DIAGNOSE
PROCESS FOR THE PREPARATION OF FLUORESCENT BORON-SUBSTITUTED DIPYRROMETHENES AND USE THEREOF FOR DIAGNOSIS

(30) Priorité: 29.12.2008 FR 0807473
(43) Date de publication de la demande: 09.11.2011
(73) Titulaire: Centre National de la Recherche Scientifique, 75016 Paris (FR); Université de Strasbourg, 67081 Strasbourg Cedex (FR)
(72) Inventeur: DE BARRY, Jean, F-67100 Strasbourg (FR); LIEGEOIS, Corinne, F-67000 Strasbourg (FR); HAEFELE, Alexandre, F-67540 Ostwald (FR); BURA, Thomas, F-57385 Teting sur Nied (FR); ULRICH, Gilles, F-67460 Souffelweyersheim (FR); ZIESSEL, Raymond, F-67460 Souffelweyersheim (FR)
(74) Mandataire: LLR
(86) Numéro de dépôt international: PCT/FR2009/001486
(87) Numéro de publication internationale: WO 2010/076433

(56) Documents cités:
- WO-A-2006/087459

### SEQUENCE LISTING

<110> CENTRE NATIONAL DE LA RECHERCHE SCIENTIFIQUE UNIVERSITE DE STRASBOURG DE BARRY Jean LIEGEOIS Corinne HAEFELE Alexandre BURA Thomas ULRICH Gilles ZIESSEL Raymond
<120> Nouveaux composés dipyrrométhènes-borosubstitués fluorescents et leur utilisation pour le diagnostic
<130> CB/es F644 221WO
<160> 2
<170> PatentIn version 3.3
<210> 1
   <211> 42
   <212> PRT
   <213> Homo sapiens
<400> 1
<210> 2
   <211> 42
   <212> PRT
   <213> Homo sapiens
<400> 2
   Ala Ile Val Val Gly Gly Val Met Leu Gly Ile Ile Ala Gly Lys Asn
   1               5                   10                  15
   Ser Gly Val Asp Glu Ala Phe Phe Val Leu Lys Gln His His Val Glu
               20                  25                  30
   Tyr Gly Ser Asp His Arg Phe Glu Ala Asp
           35                  40

## Description

La présente invention se rapporte à un procédé de préparation de nouveaux composés fluorescents dérivés non fluorés de dipyrrométhènes-bore et à leur utilisation pour le marquage fluorescent de molécules biologiques. L'invention se rapporte encore à des molécules biologiques marquées avec lesdits composés fluorescents et leur utilisation dans des méthodes de détection telles que des méthodes de diagnostic médical ; en particulier, les méthodes de détection selon l'invention s'avèrent particulièrement utiles pour le diagnostic de maladies neurodégénératives telles que la maladie d'Alzheimer.

Les marqueurs fluorescents sont très utilisés pour détecter et/ou doser des molécules biologiques dans les domaines de l'immunologie, de la biologie moléculaire, du diagnostic médical ou encore des puces à ADN.

Parmi les nombreux composés de l'art antérieur utilisables comme marqueurs fluorescents, on peut citer notamment les difluorures de dipyrrométhènes-bore (désignés ci-après par DFMB). Les brevets US 4,774,339, US 5,187,288 et US 5,451,663 décrivent des composés dérivés de DFMB contenant des groupes fonctionnels fluorescents pouvant être utilisés pour marquer des molécules biologiques ou des polymères.

Cependant, les dérivés de DFMB sont instables chimiquement ; ainsi, en milieu basique ou en présence de nucléophiles tels que des amines ou des alcoolats, les atomes de fluor de ces composés sont facilement substitués.

Les Demandes Internationales WO 2006/087459 et WO 2006/087458 décrivent des composés dérivés de dipyrrométhènes-bore non fluorés. Ces composés présentent une meilleure stabilité chimique que les dérivés DFMB ; en particulier, ils sont résistants aux conditions basiques utilisées notamment pour la déprotection des groupements de type carbamate (par exemple : Fmoc) lors des synthèses automatisées de polypeptides.

Il demeure cependant le besoin de disposer de composés fluorescents présentant :
- une stabilité chimique encore améliorée ;
- un haut rendement quantique de fluorescence et des coefficients d'extinction molaire élevés ;
- des longueurs d'onde d'excitation et d'émission pouvant être contrôlées ; et
- une fonction permettant un greffage aisé sur des molécules biologiques.

C'est ce à quoi est parvenue la Demanderesse en mettant au point de nouveaux composés fluorescents de formules générale (I) qui sont des dérivés non fluorés de dipyrrométhènes-bore présentant une fonction carbonyle permettant leur greffage sur des molécules biologiques. Les composés de formule générale (I) sont plus stables chimiquement que les fluorophores traditionnels comme la fluorescéine, les rhodamines et les difluoroboradiazaindacènes ; en outre, ces composés sont résistants aux différents réactifs utilisés lors de la synthèse sur support solide de peptides ou d'oligonucléotides et sont donc particulièrement adaptés au marquage des acides aminés ou des nucléotides.

Dans le cadre de l'invention, on entend par molécule biologique un acide aminé, un polypeptide, une protéine, la biotine ou ses dérivés ou analogues structuraux, un nucléotide ou un acide nucléique (ARN, ADN).

Un fluorophore (aussi désigné par composé ou marqueur fluorescent) est un composé comportant un groupement fonctionnel capable d'absorber l'énergie lumineuse à une longueur d'onde (dite longueur d'onde d'excitation ou d'absorption) et de restituer toute ou partie de l'énergie absorbée par une émission lumineuse à une longueur d'onde (dite longueur d'onde d'émission) égale ou plus grande que la longueur d'onde d'absorption ; un fluorophore peut être lié de façon covalente à une molécule telle qu'une molécule biologique.

De façon générale, une molécule est dite marquée lorsqu'elle comporte au moins un atome ou un groupement d'atomes détectable tel qu'un atome ou un groupement radioactif, un chromophore ou un fluorophore.

Dans le cadre de la présente invention, à moins qu'il n'en soit précisé autrement, une molécule est dite marquée lorsqu'elle est liée de façon covalente à un fluorophore.

On entend par marquage le procédé consistant à lier de façon covalente un fluorophore à une molécule biologique ; de préférence, la molécule biologique est un acide aminé ou un nucléotide, le fluorophore étant alors couplé sur la chaîne latérale de l'acide aminé ou du nucléotide.

Une fonction carbonyle est une fonction de structure suivante : 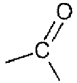 présente par exemple dans un acide carboxylique, un ester, un amide ou un thioester. Dans le cadre de la présente invention, la fonction carbonyle des composés de formule générale (I) est définie par le groupement -(Ar)ₘ-CO-Z où Ar, m et Z sont définis ci-après.

Les dérivés non fluorés de dipyrrométhènes-bore présentant une fonction carbonyle permettant leur greffage sur des molécules biologiques sont des composés de formule générale (I) : 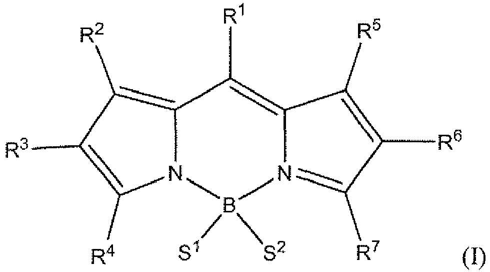 dans laquelle :
- R¹ est choisi dans le groupe constitué par -Ar-CO-Z ; un atome d'hydrogène (-H) ; -L-H ; -G et -L-G ;
   où Ar, Z, L et G sont définis ci-après ;
- **R³, R⁴, R⁶ et R⁷** sont choisis indépendamment les uns des autres dans le groupe constitué par -(Ar)ₘ-CO-Z ; un atome d'hydrogène (-H) ; -L-H ; -G et -L-G ;
   où m est 0 ou 1 ;
   étant entendu qu'un seul des substituants R¹, R³, R⁴, R⁶ et R⁷ est -Ar-CO-Z ou - (Ar)ₘ-CO-Z ; préférentiellement R¹ est -Ar-CO-Z ;
- **R²** et **R⁵** identiques ou différents sont choisis indépendamment l'un de l'autre dans le groupe constitué par un atome d'hydrogène (-H) ; -L-H ; -G et -L-G ;
   où L et G sont définis ci-après ;
- **S¹** et **S²** sont des groupements hydrophiles, identiques ou différents, de formule -C≡C-L'-A ;
   dans laquelle L' et A sont définis ci-après ;
- **Ar** est choisi parmi un arylène ou un hétéroarylène en C₅-C₁₄ sur lequel le groupe -CO-Z est en position ortho, méta ou para, de préférence para ; Ar est préférentiellement un groupe benzène, naphtalène, anthracène, pyrène, pyridine, pyrimidine, thiophène ou pyrrole ;
- **Z** est un groupement permettant le greffage du composé de formule générale (I) sur une molécule biologique ; en particulier, Z est choisi parmi un groupement -OH, -O-succinimide, -O-maléimide, -N-glycine, -N-lysine, -Y-L"-NH₂, -Y-L"-COOH, -Y-L"-SH où **Y** est choisi parmi les atomes de N et O et **L"** est défini ci-après ;
- **L** et **L"** sont choisis indépendamment l'un de l'autre parmi une liaison simple ; une chaîne carbonée saturée éventuellement ramifiée en C₁-C₁₀, de préférence en C₁-C₆ ; un arylène en C₆-C₁₆ sur lequel les groupements -H ou -G pour L et -NH₂, -COOH ou -SH pour L" sont en position ortho, méta ou para, de préférence para ; un alkénylène en C₂-C₄ ; un alkynylène en C₂-C₄ ; une chaîne carbonée linéaire ou ramifiée en C₁-C₂₀ interrompue par 1 à 10 atomes d'oxygène ; une chaîne carbonée saturée, linéaire ou ramifiée en C₁-C₂₀ interrompue par une à quatre fonctions amides - CO-NH- ; un segment nucléotidique et/ou un segment de sucres ;
- **G** est choisi dans le groupe constitué par l'ester succinimidyle, l'ester sulfosuccinimidyle, l'isothiocyanate, l'isocyanate, l'iodoacétamide, la maléimide, les halosulfonyles, les phosphoramidites, les alkylimidates en C₂-C₅, les arylimidates en C₆-C₁₀, les halogénoacides, de préférence, les chloroacides et les bromoacides, les hydrazines substituées par un alkyle en C₁-C₄, les hydroxylamines substituées par un alkyle en C₁-C₄, les carbodiimides et les perfluorophénols ;
- **L'** est choisi parmi une liaison simple ; un alkénylène en C₁-C₁₀ ou une chaîne carbonée saturée, linéaire ou ramifiée, en C₁-C₂₀ interrompue par 1 à 10 atomes d'oxygène ;
- **A** représente un groupement choisi parmi un alkyle en C₁-C₄, de préférence, le méthyle ; un groupement phosphate ou un groupement sulfonate.

De façon préférée, A est un méthyle, un propyl sulfonate, un éthyl sulfonate ou un méthylphosphate.

Les groupements arylènes en C₆-C₁₆ préférés sont choisis parmi le benzène, le naphtalène et l'anthracène.

Par les termes « alkyle en C₁-C₄ », on désigne dans l'invention un radical hydrogénocarboné linéaire ou ramifié ou un cycloalkyle comprenant de 1 à 4 atomes de carbone ; on peut citer par exemple un méthyle, un propyle, un n-butyle, un isopropyle...

Par « alkénylène en C₂-C₄ », on entend dans l'invention une chaine carbonée linéaire de 2 à 4 atomes de carbone comprenant une double liaison entre deux atomes de carbone.

Par « alkynylène en C₂-C₄ », on entend dans l'invention une chaine carbonée linéaire de 2 à 4 atomes de carbone comprenant une triple liaison entre deux atomes de carbone.

Par « alkénylène en C₂-C₁₀ », on entend dans l'invention une chaine carbonée linéaire de 2 à 10 atomes de carbone comprenant au moins une double liaison entre deux atomes de carbone.

De préférence, dans le cadre de l'invention, la chaîne carbonée saturée, linéaire ou ramifiée en C₁-C₂₀ interrompue par 1 à 10 atomes d'oxygène est un poly(oxyde d'éthylène) ou un poly(oxyde de propylène) dont le motif se répète entre une et six fois.

Par segment nucléotidique, on entend une chaîne linéaire comprenant un ou plusieurs nucléotides.

Par segment de sucre, on entend une chaîne linéaire comprenant un ou plusieurs oses.

Le choix des radicaux R¹ à R⁷ permet de modifier les propriétés du composé, telles que, par exemple, sa longueur d'onde d'émission de fluorescence, son rendement quantique de fluorescence, sa solubilité et son moment dipolaire.

Une famille particulière de dérivés non fluorés de dipyrrométhènes-bore présentant une fonction carbonyle permettant leur greffage sur des molécules biologiques comprend les composés de formule générale (I) symétriques, c'est-à-dire dans lesquels R² et R⁵ sont identiques, R³ et R⁶ sont identiques, R⁴ et R⁷ sont identiques, S¹ et S² sont identiques ; auquel cas la fonction carbonyle -Ar-CO-Z est portée par R¹.

Par la suite, les composés de formule générale (I) sont également désignés par la formule P-(Ar)ₘ-CO-Z où P représente l'ensemble du composé de formule générale (I) à l'exception du substituant R¹ ou R³ ou R⁴ ou R⁶ ou R⁷ qui est le groupement -Ar-CO-Z ou -(Ar)ₘ-CO-Z ; et où Ar, m et Z ont les mêmes définitions que ci-avant.

Les composés de formule générale (I), en plus de permettre un greffage aisé sur des molécules biologiques, présentent de nombreux avantages :
- ils émettent à une forte intensité de fluorescence et ils ne perdent pas de cette intensité lorsqu'ils sont fixés sur une molécule biologique telle qu'une protéine ;
- ils sont chimiquement résistants aux étapes de synthèse sur support solide lors de la préparation de polypeptides ou de polynucléotides ;
- ils ne perturbent pas les propriétés des molécules biologiques sur lesquelles ils sont fixés.

La synthèse de composés P-(Ar)ₘ-CO-Z selon l'invention est réalisée à partir d'un intermédiaire de synthèse, dérivé de dipyrrométhènes-bore. Cet intermédiaire de synthèse est obtenu en mettant à réagir, avec le composé difluoré DFMB, un réactif organométallique (organomagnésien ou organolithien) de Grignard de formule S¹-MgX, S¹-LiX, S²-MgX ou S²LiX, où X est un atome d'halogène, dans les conditions décrites dans la Demande Internationale PCT WO 2006/087459 ; cette réaction permet d'introduire les groupements S¹ et S² dans le composé difluoré DFMB.

Cet intermédiaire de synthèse a pour formule P-(Ar)ₘ-Q ; où **P, m** et **Ar** sont tels que définis pour la formule (I) et **Q** est choisi parmi un atome d'halogène, en particulier, I, Br ou Cl; un groupement -O-triflate (le triflate a pour formule -O-SO₂-CF₃ et est aussi désigné par Tf); un groupement -O-tosylate (le tosylate a pour formule -O-SO₂-C₆H₆-CH₃ et est aussi désigné par Ts) ou un groupement -O-mésylate (le mésylate a pour formule -O-SO₂-CH₃ et est aussi désigné par Ms).

A partir de cet intermédiaire de synthèse P-(Ar)ₘ-Q, le radical -Q est transformé en fonction carbonyle par réaction avec une source de monoxyde de carbone ou du monoxyde de carbone, en présence :
- d'un nucléophile choisi selon la nature de la fonction carbonyle souhaitée : de l'eau pour obtenir un acide carboxylique ; un alcool pour obtenir un ester ; une amine pour obtenir un amide et un thiol pour obtenir un thioester ; et
- d'un catalyseur à base de palladium, par exemple le Pd(PPh₃)₂Cl₂.

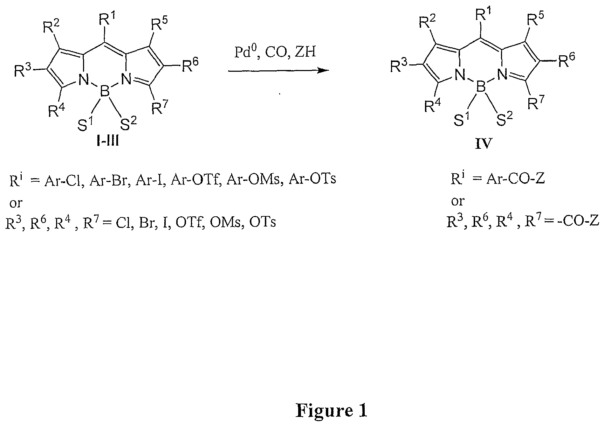

Le schéma de cette synthèse est représenté sur la **Figure 1****.**

Le monoxyde de carbone utilisé dans cette réaction peut être non marqué ; marqué avec un atome de ¹³C avec un taux de marquage contrôlable de 1 à 99 % pour un suivi RMN, ou marqué avec un atome ¹⁴C pour un suivi radioactif ; un tel marquage permet un contrôle supplémentaire des molécules biologiques marquées par le composé (I) fluorescent selon l'invention.

Il est nécessaire d'introduire la fonction carbonyle -CO-Z sur l'intermédiaire de synthèse P-(Ar)ₘ-Q comportant déjà les groupements S¹ et S² sur le bore ; en effet, la présence de fonction carbonyle n'est pas compatible avec l'utilisation d'un composé organométallique nécessaire à l'ajout de S¹ et S².

Un exemple particulier de préparation de composés de formule générale (I) où la fonction carbonyle est en R¹ est illustré à l'exemple 1.

De manière similaire des fonctions carbonyles peuvent être introduites directement en position R³ ou R⁴ ou R⁷ ou R⁶ si ces positions possèdent un groupement -Q tel que défini ci-avant, par l'intermédiaire d'un couplage catalysé par le palladium en présence de monoxyde de carbone ou de toute autre source de monoxyde de carbone.

Ainsi un objet de la présente invention se rapporte à un procédé de préparation de composés de formule générale (I), caractérisé en ce qu'il comprend la transformation de l'intermédiaire de synthèse **P-(Ar)ₘ-Q** en composé de formule générale (I) par réaction avec du monoxyde de carbone en présence d'un nucléophile choisi parmi l'eau, un alcool, une amine ou un thiol et d'un catalyseur au palladium ; ledit intermédiaire de synthèse est tel que **P** est un groupement de structure identique au composé de formule générale (I) à préparer à l'exception du radical R¹ ou R³ ou R⁴ ou **R**⁶ ou **R**⁷ selon celui qui est le groupement -Ar-CO-Z pour R¹ ou -(Ar)ₘ-CO-Z pour R³ ou R⁴ ou R⁶ ou R⁷ ; **Ar** est tel que défini pour la formule (I), **m** est 0 ou 1 étant entendu que m est 1 si R¹ est la fonction carbonyle -Ar-CO-Z et **Q** est choisi parmi un atome d'halogène ; un -O-triflate ; un -O-tosylate ou un -O-mésylate. Dans un mode de réalisation privilégié, R¹ est -Ar-CO-Z.

La présente invention a encore pour objet l'utilisation d'un composé de formule générale (I) comme marqueur fluorescent.

Grâce à sa fonction carbonyle, le composé de formule (I) peut aisément être greffé sur une molécule biologique telle qu'un acide aminé, une protéine, la biotine ou un de ses dérivés ou analogue structuraux ou un nucléotide.

Ainsi, selon un autre de ses objets, la présente invention se rapporte à une molécule biologique marquée de formule générale (II) (ci-après désignée par « molécule biologique marquée ») :

P-(Ar)ₘ-CO-(X)ₙ-T (II)

dans laquelle :
- **P, Ar** et **m** sont tels que définis ci-avant ; m est 0 ou 1 étant entendu que m est 1 si le groupement -(Ar)ₘ-CO-(X)ₙ-T est substitué à R¹ ;
- **X** est un espaceur porteur d'une fonction carboxylique, amine ou thiol ; il est par exemple une chaîne comprenant entre un et trois acides aminés, ou encore un alkylène en C₁-C₆ pouvant être interrompu par 2 ou 3 atomes d'oxygène et se liant de façon covalente au fluophore et à la molécule biologique par l'intermédiaire d'une fonction amide, éther, ester ou thioester ou d'un pont disulfure ;
- **n** est un nombre entier égal à 0 ou 1 ;
- **T** est une molécule biologique.

Dans le cadre de l'invention, l'espaceur sert à éloigner la molécule biologique T du fluorophore P. De préférence, l'espaceur est inerte chimiquement, c'est-à-dire qu'il ne réagit avec aucun des groupements qu'il éloigne ; en particulier, il n'affecte pas la fluorescence du fluorophore ni l'activité biologique de la molécule biologique.

En tant que molécule biologique, T est choisi parmi un acide aminé naturel ou synthétique, un polypeptide, une protéine, la biotine ou ses dérivés ou analogues structuraux, un nucléotide ou un acide nucléique (ARN, ADN).

Lorsque T est un acide aminé, il est choisi parmi l'alanine, l'arginine, l'asparagine, l'aspartate ou l'acide aspartique, la cystéine, le glutamate ou l'acide glutamique, la glutamine, la glycine, l'histidine, l'isoleucine, la leucine, la lysine, la méthionine, la phénylalanine, la proline, la sérine, la thréonine, le tryptophane, la tyrosine et la valine. De préférence, T est la lysine ou la glycine.

Lorsque T est un nucléotide, il est choisi parmi les ribonucléotides tels que l'adénosine, l'uridine, la guanosine, la cytidine ou la ribothymidine ou les désoxyribo-nucléotides tels que la désoxyadénosine, la désoxyuridine, la désoxyguanosine, la désoxycytidine ou la désoxyribothymidine.

Les molécules biologiques marquées sont préparées selon les méthodes qui suivent.

Lorsque T est un acide aminé ou une protéine, la préparation de la molécule biologique marquée de formule générale (II) est réalisée à partir d'un composé de formule générale (I) fonctionnalisé avec un groupement Z porteur d'un acide carboxylique ; ledit composé (I) est (i) transformé en ester d'hydroxysuccinimide ou de tétrafluorophényle puis (ii) mis à réagir avec l'acide aminé ou la protéine (Guide to Labeling proteins with Fluorescent Dyes - Note 7.1 http://www.invitrogen.com/site/ us/en/home/References/Molecular-Probes-The-Handbook/).

Le couplage peptidique peut également être réalisé avec du 1-(3-diméthylaminopropyl)-3-éthylcarbodiimide (EDCI, voir l'exemple 1).

Il peut être avantageux d'utiliser à l'étape (ii) un acide aminé déjà lié à un groupement protecteur du type BOC (t-Butoxycarbonyl), Fmoc (9-Fluorényl-méthyloxycarbonyl), Bpoc (2-(4-biphénylyl) propyl(2)oxycarbonyl), Nps (2-nitro-phénylsulfényl) ou Dts (dithia-succionyl), le groupement protecteur étant introduit sur la fonction amine de l'acide aminé qui est impliquée dans la formation du polypeptide ; en effet, ainsi protégé, l'acide aminé marqué peut ensuite être utilisé dans une technique classique de synthèse de peptides en phase solide (Merrifield, R. B. J. Am. Chem. Soc. 1963, 85, 2149-2154). A l'issue de cette synthèse, on obtient un polypeptide fluorescent grâce à la présence du fluorophore sur au moins un de ses acides aminés.

Selon une variante de l'invention, T est la biotine ou un de ses dérivés ou analogues structuraux. Dans ce cas, la préparation de la molécule biologique marquée de formule générale (II) est réalisée à partir d'un composé de formule générale (I) fonctionnalisé avec un groupement Z porteur d'un acide carboxylique ; ledit composé (I) est (i) transformé en amide par une réaction d'hydrolyse avec une diamine aliphatique pouvant être le 1,6-diaminohexane, le 1,3-diaminopropane ou le 1,2-diaminoéthane puis (ii) mis à réagir, par toute méthode classique connue de l'homme du métier, avec la biotine ou un de ses dérivés ou analogues structuraux possédant une fonction acide carboxylique libre.

Selon une autre variante de l'invention, T est un nucléotide. Alors la préparation de la molécule biologique marquée de formule générale (II) est réalisée à partir d'un composé de formule générale (I) fonctionnalisé avec un groupement Z porteur d'un acide carboxylique ; ledit composé (I) est mis à réagir, par toute méthode classique connue de l'homme du métier, avec un nucléotide modifié avec une fonction amine libre.

Ce nucléotide marqué est ensuite avantageusement utilisé pour la synthèse d'oligonucléotides marqués.

Il existe plusieurs techniques de synthèse d'oligonucléotides ; on peut notamment citer la synthèse oligonucléotidique sur support solide qui s'inspire de celle proposée par Merrifield en synthèse peptidique ou encore la synthèse d'oligonucléotides par la méthode au phosphoramidite (Matteucci, M. D.; Caruthers, M. H. Tetrahedron Lett. 1980, 21, 719-722 ; Matteucci, M. D.; Caruthers, M. H. J. Am. Chem. Soc. 1981, 103, 3185-3191).

L'interaction de la molécule biologique marquée avec un ligand conduit à une modification du spectre d'émission de fluorescence de ladite molécule ; de cette propriété, découle la mise au point de méthodes de détection.

Un autre avantage découlant de cette propriété est que la mise en oeuvre des méthodes de détection peut se faire uniquement avec le fluorophore selon l'invention et ne nécessite pas d'association de plusieurs fluorophores ou de couple fluorophore-suppresseur de fluorescence comme c'est par exemple le cas pour des techniques telles que la technique FRET de mesure d'interaction entre des protéines (pour Fluorescence Resonance Energy Transfer, voir Lopper et al., Protein-protein interactions : identification, 2007, Encyclopedia of Life Science) ou la PCR en temps réel (Poitras et al., La PCR en temps réel : principes et applications, 2002, Reviews in Biology and Biotechnology, vol. 2, N°2, p.2-11) ; cela n'empêche cependant pas d'utiliser les fluorophores selon l'invention dans ces techniques.

Dans le cadre de l'invention, un ligand est une molécule dont on souhaite détecter la présence ou non dans un échantillon ; la molécule biologique est choisie pour sa propriété d'interagir avec le ligand.

Le spectre d'émission d'un fluorophore peut être mesuré par toute méthode classique connue de l'homme du métier. De préférence, le spectre d'émission de fluorescence sera mesuré à l'aide d'un spectrofluorimètre ou d'un dispositif de microscopie équipé d'un détecteur spectral ; le spectre est représenté sur un graphe avec la longueur d'onde sur l'axe des abscisses et l'intensité lumineuse sur l'axe des ordonnées.

Il est donc possible de mesurer l'indice d'anisotropie du spectre de fluorescence d'une molécule biologique marquée dans différentes conditions (en présence ou non d'échantillon à tester).

Par indice d'anisotropie d'un spectre (aussi dénommé indice spectral ou indice de déformation), on entend un indice représentatif de l'asymétrie du spectre par rapport à la position du pic principal d'émission de fluorescence.

Pour déterminer l'indice d'anisotropie des spectres mesurés, les spectres sont soumis à une analyse qui consiste à les décomposer en courbes Gaussiennes élémentaires par déconvolution en utilisant une méthode de régression non-linéaire. Les caractéristiques de ces courbes Gaussiennes (position du pic, amplitude, aire et dispersion) sont calculées :
- La position du pic correspond à la longueur d'onde à laquelle on observe un maximum d'intensité lumineuse ;
- L'amplitude est l'intensité lumineuse du pic ;
- L'aire est l'intégrale de l'intensité lumineuse sous la courbe ;
- La dispersion est la largeur à mi-hauteur de la courbe exprimée en longueur d'onde.

Au sein d'un même spectre, le rapport de l'amplitude ou de l'aire des deux principales Gaussiennes permet de définir l'indice de déformation du spectre de fluorescence en fonction des conditions d'enregistrement du spectre. Les variations de la valeur de l'indice de déformation du spectre mesuré en présence ou non d'un échantillon permettent de détecter la présence d'un ligand et d'en évaluer la proportion.

Dans une variante, l'anisotropie du spectre peut également être mesurée à l'aide de filtres optiques à bande passante. L'indice de déformation du spectre sera alors évalué par le rapport des intensités de fluorescence mesurées à l'aide des différents filtres à bande passante.

Ainsi, selon encore un autre de ses objets, l'invention se rapporte à une méthode de détection du ligand d'une molécule biologique marquée de formule générale (II) dans un échantillon comprenant les étapes suivantes :
a) mesure du spectre d'émission de fluorescence de l'échantillon à tester seul ; le spectre obtenu est désigné « ligne de base » ;
b) mesure du spectre d'émission de fluorescence de la molécule biologique marquée en solution ; soustraction de la ligne de base au spectre obtenu et calcul de l'indice de déformation ;
c) incubation de ladite molécule biologique marquée en solution avec l'échantillon à tester et obtention d'un mélange ;
d) mesure du spectre d'émission de fluorescence du mélange obtenu à l'étape c) ; soustraction de la ligne de base au spectre obtenu et calcul de l'indice de déformation ;
e) comparaison des indices de déformation calculés aux étapes b) et d) et détection de l'interaction entre le ligand et la molécule biologique marquée lorsque lesdits indices sont différents.

De préférence, à l'étape b), la molécule biologique est en solution dans une solution qui a la même composition que la solution dans laquelle on mesure le spectre de fluorescence de la molécule marquée en présence de l'échantillon biologique.

Selon une variante de l'invention, la méthode de détection permet également de quantifier le ligand dans l'échantillon. Pour ce faire, il est préalablement réalisé une courbe d'étalonnage de la valeur de l'indice de déformation du spectre en fonction de la quantité de ligand.

En outre, si le ligand peut présenter des conformations différentes -ce qui peut être le cas pour un peptide dont la conformation tridimensionnelle peut varier en fonction de son environnement-, le spectre de fluorescence de la molécule biologique marquée complexée au ligand sera différent selon la conformation du ligand ; cette propriété permet de mettre en oeuvre une méthode de détection qualitative d'un ligand.

L'échantillon peut être d'origine biologique, c'est-à-dire qu'il représente tout ou partie d'un organe, d'un tissu, d'une cellule, d'un microorganisme...

L'échantillon peut également être de toute autre nature, il peut par exemple et sans aucun caractère limitatif, être un aliment dans lequel on veut contrôler l'absence de contaminants tels que des microorganismes indésirables ou même pathogènes, des toxines, des polluants comme des pesticides...

La mise en oeuvre de la méthode de détection selon l'invention peut également être utile pour diagnostiquer une maladie.

A ce titre, il a été mis au point une méthode de diagnostic de maladies neurodégénératives telles que la maladie d'Alzheimer.

L'accroissement général de la longévité favorise la prévalence de maladies neurodégénératives et, en particulier, de la maladie d'Alzheimer. Ces pathologies constituent un problème social et économique majeur pour la société moderne. L'examen clinique associé à l'imagerie médicale et à des tests neuropsychologiques permettent un diagnostic tardif dans la phase terminale de la maladie et le diagnostic définitif ne peut être obtenu que par l'examen post mortem de tissus cérébraux, ce qui n'est pas satisfaisant. Ainsi, différentes voies ont été explorées pour identifier un marqueur biologique périphérique qui permettrait un diagnostic non invasif de la maladie.

Il a été mis en évidence que les érythrocytes sont altérés dans la maladie d'Alzheimer ; cette altération se manifeste par une conformation anormale de la protéine kinase C qui peut être provoquée par la dérégulation de la concentration calcique intracellulaire (Janoshazi et al. Neurobiol. Aging 2006, 27 : 245-251).

En outre, il a été montré que le peptide bêta-amyloïde humain, produit dans les tissus nerveux et dans le sang au cours de la maladie d'Alzheimer, pouvait interagir avec la surface des érythrocytes (Mattson MP et al. Brain Res 1997, 771: 147-153) en modifiant l'homéostasie calcique.

Les essais menés dans le cadre de l'invention montrent que les cellules vivantes, telles que les érythrocytes, préalablement placées en présence de faibles concentrations de peptide bêta-amyloïde 1-42 non marqué, sont capables de lier subséquemment un peptide bêta-amyloïde porteur d'un fluorophore décrit dans l'invention ; cette liaison s'accompagne d'une déformation spécifique du spectre de fluorescence du fluorophore permettant d'évaluer la concentration de peptide bêta-amyloïde 1-42 non marqué à laquelle les cellules ont été préalablement exposées. Il est donc possible de quantifier le peptide bêta-amyloïde 1-42 humain circulant chez un individu.

Plus spécifiquement, la méthode de diagnostic de la maladie d'Alzheimer met en oeuvre la méthode de détection selon l'invention ; elle permet d'évaluer la quantité de peptide bêta-amyloïde 1-42 dans un échantillon où :
- l'échantillon biologique contient des érythrocytes, par exemple, du sang ; et
- la molécule biologique marquée est telle que T est un peptide dérivé du peptide bêta-amyloïde 1-42.

Le peptide bêta-amyloïde 1-42 est le peptide de séquence SEQ ID NO:1.

Par dérivé du peptide bêta-amyloïde 1-42, on entend un peptide dont un ou plusieurs des acides aminés ont été substitués par le même acide aminé marqué par un composé fluorescent de formule générale (I) selon l'invention ; ce dérivé peut également comprendre une ou plusieurs insertions d'un acide aminé marqué à tout niveau de sa chaîne.

A titre d'exemple, la préparation d'un dérivé du peptide bêta-amyloïde 1-42 est décrit à l'exemple 1 et conduit au peptide suivant :
H-Asp-Ala-glu-Phe-Arg-His-Asp-Ser-Gly-Tyr-Glu-Val-His-His-Gln-**Lys***-Leu-Val-Phe-Phe-Ala-Glu-Asp-Val-Gly-Ser-Asn-Lys-Gly-Ala-Ile-Ile-Gly-Leu-Met- Val-Gly-Gly-Val-Val-Ile-Ala-OH (SEQ ID NO:1) où le fluorophore est porté par la lysine en position 16.

D'autres acides aminés que la lysine peuvent être marqués et utilisés pour préparer le dérivé du peptide bêta-amyloïde 1-42 ; il s'agit de préférence des acides aminés présentant une fonction amine ou acide carboxylique sur leur chaîne latérale.

La valeur des indices de déformation des spectres de fluorescence mesurés dans l'échantillon à tester puis la comparaison avec une courbe d'étalonnage permet d'évaluer la proportion de peptide bêta-amyloïde 1-42 présent dans l'échantillon à tester.

Pour la mise en oeuvre de cette méthode de diagnostic, la courbe d'étalonnage est réalisée en utilisant des érythrocytes humains provenant d'un sujet sain et préincubés pendant 18 heures en présence de concentrations croissantes de peptide bêta-amyloïde 1-42. Alors l'étape e) de la méthode selon l'invention consiste à comparer l'indice de déformation du spectre obtenu à l'étape d) avec la courbe d'étalonnage.

Ainsi, cette méthode permet d'évaluer la quantité de peptide bêta-amyloïde 1-42 circulante tout en s'affranchissant des difficultés de dosage induites par l'interaction de ce peptide avec d'autres protéines solubles telles que l'albumine sérique. Elle constitue un moyen original de diagnostic de la maladie d'Alzheimer simple, rapide et peu coûteux. C'est également un moyen de pronostic, de suivi de l'efficacité d'un traitement contre la maladie et de développement d'agents thérapeutiques à l'aide de lignées cellulaires ou de modèles animaux de la maladie.

Une autre variante de mise en oeuvre de la méthode selon l'invention permet l'identification d'un composé capable d'interagir avec le ligand d'une molécule biologique marquée, une telle variante comprend les étapes suivantes :
a) mesure du spectre d'émission de fluorescence et calcul de l'indice de déformation de la molécule biologique marquée en solution ;
b) incubation de ladite molécule biologique marquée en solution avec un ligand et obtention d'un mélange 1 ;
c) mesure du spectre d'émission de fluorescence et calcul de l'indice de déformation du mélange 1 ;
d) incubation dudit ligand avec ledit composé à tester puis ajout de ladite molécule biologique marquée en solution et obtention d'un mélange 2 ;
e) mesure du spectre d'émission de fluorescence et calcul de l'indice de déformation du mélange 2 ;
f) détection de l'interaction entre le ligand et le composé à tester par comparaison des indices de déformation calculés aux étapes a), c) et e).

Ainsi mise en oeuvre, la méthode permet d'identifier de potentiels agents thérapeutiques, ou encore de mesurer l'efficacité d'un tel agent *in vitro* et *ex vivo.*

L'invention concerne également des trousses diagnostiques ou des ensembles de réactifs pour la mise en oeuvre de la méthode de détection selon l'invention. Ces trousses sont caractérisées en ce qu'elles comprennent au moins une molécule biologique marquée de formulé générale (II) selon l'invention, avec les contenants et réactifs appropriés et un manuel d'utilisation.

L'invention est maintenant décrite plus en détail au regard des figures et des exemples qui suivent. Il doit être bien entendu, toutefois, que ces exemples sont donnés uniquement à titre d'illustration de l'objet de l'invention, dont ils ne constituent en aucune manière une limitation.
- La **Figure 1** représente un schéma de synthèse des composés de formule générale (I).
- La **Figure 2** représente le schéma de synthèse conduisant aux composés **la-d** à **6a** tels que décrits dans l'exemple 1 (TEA = triéthylamine).
- La **Figure 3** représente le schéma de synthèse conduisant aux composés **6a¹³-b et 7a, 7a¹³ et b** tels que décrits dans l'exemple 1 (EDCI = N-(3-Diméthyl-aminopropyl)-N'-éthylcarbodiimide et NHS = N-hydroxysuccinimide).
- La **Figure 4** illustre la mesure de spectres d'émission de fluorescence dans les conditions de l'exemple 3. Le graphe **A** représente le spectre de fluorescence du peptide bêta-amyloïde couplé au composé **7a** et le graphe **B** représente la déconvolution du spectre de fluorescence du peptide bêta-amyloïde couplé au composé **7a** enregistré en présence de cellules PC12 avant préincubation (tracés continus) et après préincubation (tracés en pointillés) des cellules avec le peptide bêta-amyloïde 1-42 non marqué.
- La **Figure 5** est le graphe représentant les valeurs de l'indice spectral du composé 7a couplé au peptide bêta-amyloïde 1-42 après préincubation de différentes concentrations de peptide bêta-amyloïde (Ab) non marqué comme décrit à l'exemple 4.
- La **Figure 6** est un histogramme qui illustre la variation de la valeur de l'indice spectral du peptide bêta-amyloïde 1-42 couplé au composé 7a en présence d'érythrocytes de rat préincubés avec le peptide bêta-amyloïde 1-42 non marqué comme décrit à l'exemple 5.

### Exemple 1 - préparation de composés de formule générale (I) où R¹ est - Ar-CO-Z par le procédé selon l'invention

Les composés **la-d** à **7a-b** de cet exemple ont été choisis pour leur correspondance spectrale avec les fluorophores courants les plus utilisés avec l'indice **a** : fluorescéine, indice **b :** Rhodamine 6G ; indice **c :** TMR (tétraméthyl rhodamine); indice **d** : TOTO-3.

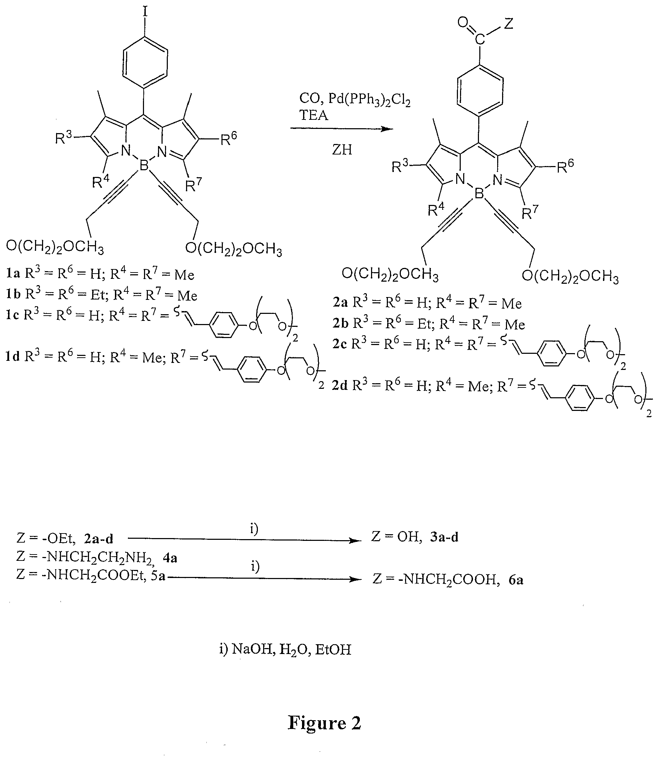

Le schéma de synthèse conduisant aux composés **la-d** à **6a** est représenté à la **Figure 2****.**

Les composés **la-d** sont obtenus par action de l'organométallique méthoxyéthoxyéthynylé (réactif de Grignard), dans le THF à 60°C sur le produit difluoré correspondant (pouvant être préparé comme décrit dans le Brevet US 4,744,339) en suivant les modes opératoires décrits dans la Demande Internationale WO 2006/087459.

L'iode porté par les composés **la-d** est ensuite converti en groupement carbonyle sous atmosphère de monoxyde de carbone en présence de catalyseur Pd(PPh₃)₂Cl₂, dans un mélange de triéthylamine benzène à 70°C.

Le choix du nucléophile utilisé permet d'obtenir directement de nombreuses fonctions pouvant être ensuite utilisées pour le couplage sur une molécule biologique :
- avec de l'éthanol, les ester éthyliques **2a-d** sont obtenus avec de très bons rendements ;
- en utilisant une diamine aliphatique en excès, des composés de type **4** peuvent être obtenus ;
- lorsqu'on utilise directement un acide aminé avec une amine libre et protégé au niveau de la fonction acide carboxylique (par exemple, un éthylester de glycine), l'acide aminé peut être introduit directement pour donner des composés de type **5.**

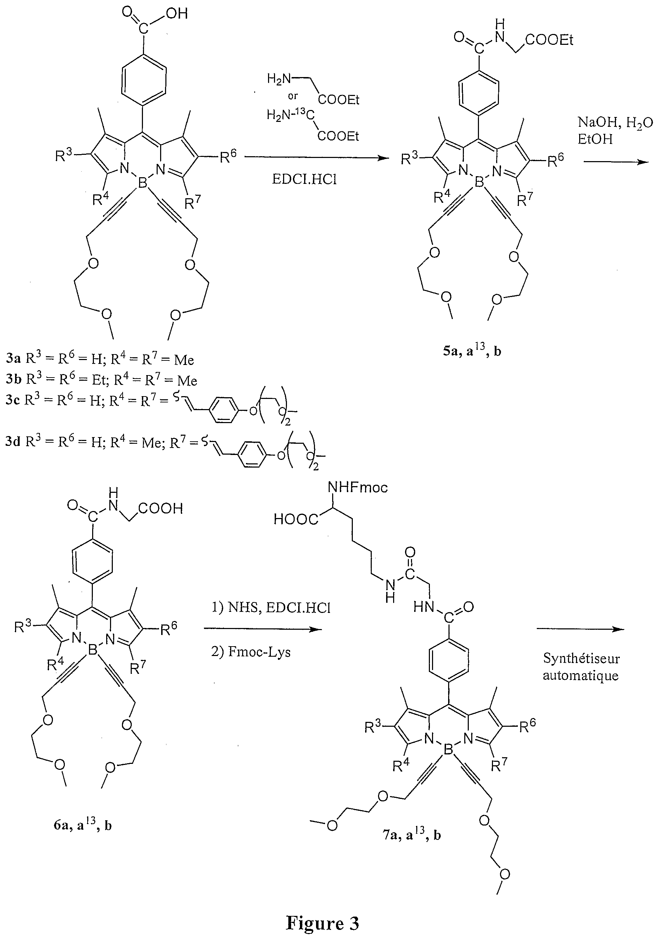

A partir des composés **3a-d,** on obtient les composés **7a, a¹³, b** selon le schéma de synthèse représenté en **Figure 3****.** Les composés portant un exposant « ¹³ » sont marqués au ¹³C.

La fonction ester des composés **2a-d** peut être saponifiée pour obtenir les acides carboxyliques **3a-d** correspondants, en présence de soude dans l'éthanol (voir la **Figure 2**). Ces acides carboxyliques peuvent être soit directement utilisés pour le marquage de molécules biologiques, soit liés à des espaceurs de types acides aminés (greffés en utilisant des techniques de couplage peptidiques utilisées avec les composés **6a-b** ci-après comme représenté sur le schéma de synthèse de la **Figure 3**).

Afin de pouvoir être utilisés dans un synthétiseur de peptides, les composés **3a-b** sont couplés avec un ester de glycine via une synthèse peptidique classique (EDC, DMAP). Cette étape permet si besoin d'introduire par exemple un fragment marqué avec un atome ¹³C (composé **5a**¹³) pour permettre une traçabilité en RMN.

Les composés **5a-b** obtenus sont ensuite saponifiés en acides carboxyliques **6a-b.**

Les acides **6a-b** sont transformés en ester d'hydroxysuccinimide, qui sont directement mis à réagir avec une lysine-Fmoc.

Les composés **7a-b** peuvent être directement utilisés pour introduire un acide aminé Lys marqué dans un synthétiseur de peptide.

### Préparation du composé 2a

Le composé **2a** est préparé selon le schéma réactionnel suivant :

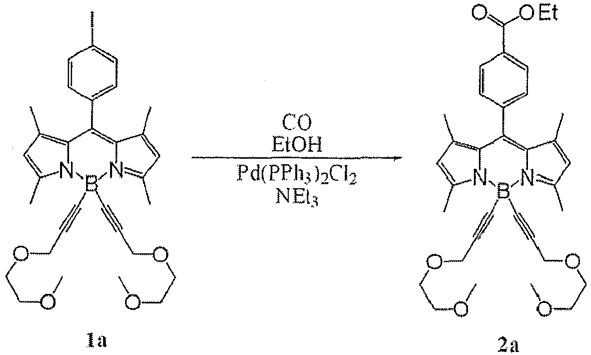

A une solution du composé **la** (235 mg, 0,37 mmol) dans 15 mL de benzène a été ajouté 1 mL d'éthanol (17,2 mmol), 44 mg de palladium bis-triphénylphosphine bis-chlorure (0,07 mmol) et 5 mL de triéthylamine. La solution a été agitée à 70°C pendant une nuit en faisant "buller" du monoxyde de carbone. Le mélange réactionnel a été extrait au dichlorométhane et lavé à l'eau (3x20 mL). La phase organique a été séchée sur du coton hydrophile et évaporée. Le résidu a été purifié par chromatographie sur colonne de gel de silice (CH₂Cl₂/MeOH 99:1 ou AcOEt/Ether de pétrole 40:60) pour donner le composé **2a** sous forme d'une poudre orange (210 mg, 97%).

### Caractérisation du composé 2a

RMN ¹H (CDCl₃, 400 MHz) : δ = 1,33 (s, 6H), 1,42 (t, 3H, ³*J* = 7,0 Hz), 2,71 (s, 6H), 3,35 (s, 6H), 3,53 (m, 4H), 3,64 (m, 4H), 4,19 (s, 4H), 4,40 (q, 2H, ³*J* = 7,0 Hz), 6,00 (s, 2H), 7,78 (AB sys, 4H, *J_{AB}* = 8,5 Hz, *vₒδ* = 300,4 Hz);
RMN ¹³C {¹H} (CDCl₃, 100 MHz,): *δ* = 14,4, 14,8, 16,1, 59,0, 59,7, 61,4, 68,4, 68,6, 71,8, 90,9, 121,8, 128,6, 129,1, 130,3, 131,0, 140,3, 140,3, 140,9, 155,6, 166,1 ;
RMN ¹¹B (CDCl₃, 128,4 MHz) : *δ* = -10,2 (s);
UV-Vis (CH₂Cl₂) λ nm (ε, M⁻¹ cm⁻¹) = 500 (90000), 366 (4900), 308 (7700);
FAB⁺ m/z : 585,2 ([M+H]⁺, 100) ;
Analyse élémentaire calculée pour C₃₄H₄₁BN₂O₆ : C, 69,86 ; H, 7,07 ; N, 4,79. Trouvé: C, 69,77 ; H, 7,04; N, 4,59.

### Préparation du composé 2b

Le composé **2b** est préparé selon le schéma réactionnel suivant :

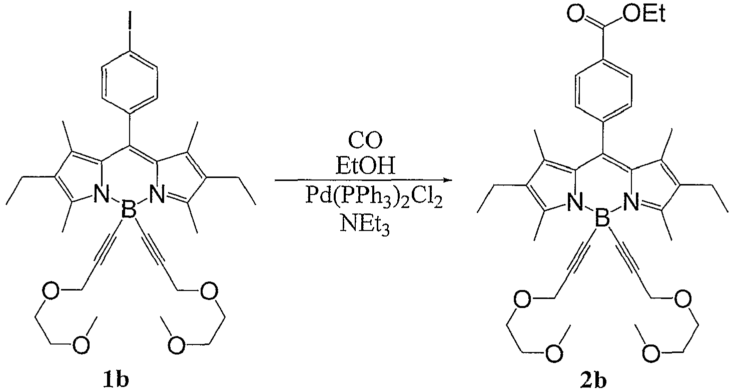

A une solution du composé **1b** (980 mg, 1,41 mmol) dans 50 mL de benzène ont été ajoutés 3 mL d'éthanol (17,2 mmol), 240 mg de palladium bis-triphénylphosphine bis-chlorure (0,07 mmol) et 15 mL de triéthylamine. La solution a été agitée à 70°C pendant une nuit en faisant "buller" du monoxyde de carbone. Le mélange réactionnel a été extrait au dichlorométhane et lavé à l'eau (3x20 mL). La phase organique a été séchée sur du coton hydrophile et évaporée. Le résidu a été purifié par chromatographie sur colonne de gel de silice (AcOEt/Ether de pétrole 20:80 ; 40:60) pour donner le composé **2b** sous forme d'une poudre orange (895 mg, quantitatif).

### Caractérisation du composé 2b

RMN ¹H (CDCl₃ 300 MHz) : 0,97 (t, 6H, ³*J* = 7,40 Hz), 1,23 (s, 6H), 1,43 (t, 3H, ³*J* = 7,10 Hz), 2,31 (q, 4H, ³*J* = 7,40 Hz) 2,69 (s, 6H), 3,35 (s, 6H), 3,53 (m, 4H), 3,65 (m, 4H), 4,19 (s, 4H), 4,43 (q, 2H, ³*J* = 7,10 Hz), 7,78 (AB sys, 4H, *J_{AB}* = 8,19 Hz, *vₒδ*= 223,07 Hz);
RMN ¹³C (CDCl₃, 300 MHz,) : *δ* =12,03, 14,07, 14,41, 14,76, 17,40, 29,78, 59,02, 59,79, 61,37, 68,57, 71,85, 90,67, 128,60, 128,95, 130,19, 130,81, 133,11, 136,02, 138,77, 141,27, 154,05, 166,28.
NMR ¹¹B (CDCl₃,128,4 MHz) : δ = -10,2 (s);
UV-Vis (CH₂Cl₂) λ nm (ε, M⁻¹ cm⁻¹) = 522 (80000),488 (20000), 381 (6400), 277 (6400).

### Préparation du composé 2c

Le composé **2c** est préparé selon le schéma réactionnel suivant :

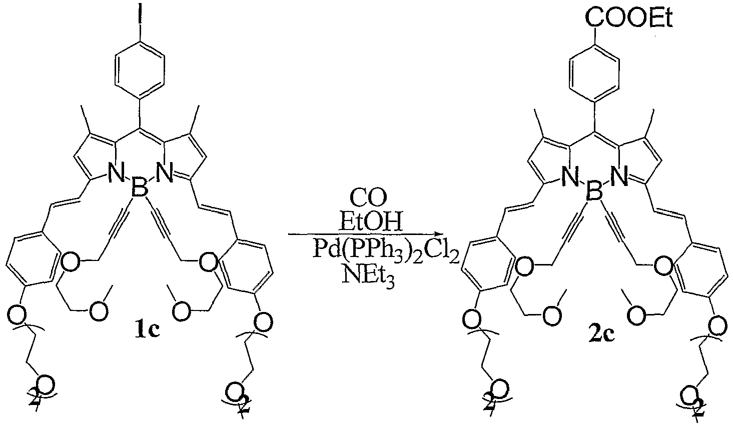

A une solution du composé **le** (100 mg, 0,095 mmol) dans 25 mL de benzène a été ajouté 1 mL d'éthanol, 24 mg de palladium bis-triphénylphosphine bis-chlorure et 5 mL de triéthylamine. La solution a été agitée à 70°C pendant une nuit en faisant "buller" du monoxyde de carbone. Le mélange réactionnel a été extrait au dichlorométhane et lavé à l'eau (3x10 mL). La phase organique a été séchée sur du coton hydrophile et évaporée. Le résidu a été purifié par chromatographie sur colonne de gel de silice (AcOEt/Ether de pétrole 80 :20 ; 100%) pour donner le composé **2c** sous forme d'une poudre bleue (88 mg, 92%).

### Caractérisation du composé 2c

RMN ¹H (CDCl₃ 200 MHz) : 1,40 (s, 6H), 1,44 (t, 3H, ³*J* = 10,5 Hz) 3,15 (m, 4H), 3,19 (s, 6H), 3,41 (s, 6H), 3,50 (m, 4H), 3,59 (m, 4H), 3,74 (m, 4H), 3,88 (m, 4H), 4,15 (s, 4H), 4,18 (m, 4H), 4,41 (q, 2H, ³*J* = 10,5 Hz), 6,62 (s, 2H), 7,27 (AB sys, 8H, *J_{AB}* = 8,73 Hz, *vₒδ* = 120,73 Hz), 7,60 (AB sys, 4H, *J_{AB}* = 16,26 Hz, *vₒδ* = 192,63 Hz) 7,83 (AB sys, 4H, *J_{AB}* = 8,34 Hz, *vₒδ* = 141,89 Hz).
UV-Vis (CH₂Cl₂) λ nm (ε, M⁻¹ cm⁻¹) = 647 (121000), 373 (72800).

### Préparation du composé 2d

Le composé **2d** est préparé selon le schéma réactionnel suivant :

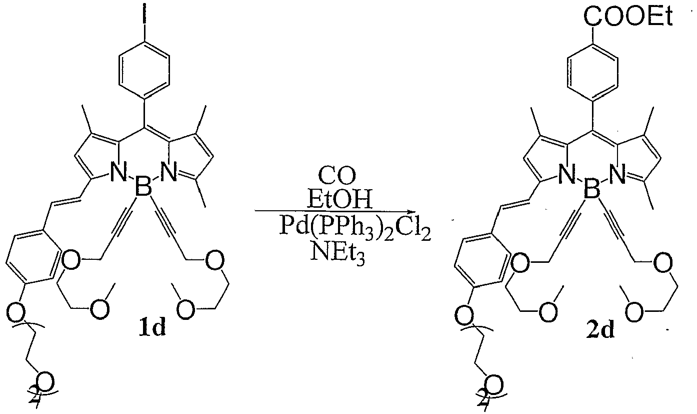

A une solution du composé **1d** (100 mg, 0,118 mmol) dans 25 mL de benzène ont été ajoutés 2 mL d'éthanol, 24 mg de palladium bis-triphénylphosphine bis-chlorure (0,07 mmol) et 5 mL de triéthylamine. La solution a été agitée à 70°C pendant une nuit en faisant "buller" du monoxyde de carbone. Le mélange réactionnel a été extrait au dichlorométhane et lavé à l'eau (3x10 mL). La phase organique a été séchée sur du coton hydrophile et évaporée. Le résidu a été purifié par chromatographie sur colonne de gel de silice (AcOEt/Ether de pétrole 20:80 ; 40:60) pour donner le composé **2d** sous forme d'une poudre violette (82 mg, 88%).

### Caractérisation du composé 2d

RMN ¹H (CDCl₃ 300 MHz): 1,35 (s, 3H), 1,38 (s, 3H), 1,43 (t, 3H, ³*J* = 7,15 Hz) 2,74 (s, 3H), 3,25 (s, 6H), 3,31 (m, 4H), 3,39 (s, 3H), 3,54 (m, 6H), 3,72 (m, 2H), 3,87 (m, 2H), 4,18 (m, 4H), 4,43 (q, 2H, ³*J* = 7 Hz), 6,02 (s, 1H), 6,59 (s, 1H), 7,22 (AB sys, 4H, *J_{AB}* = 8,67 Hz, *v*ₒ*δ*= 180,18 Hz); 7,58 (AB sys, 2H, *J_{AB}* = 16,29 Hz, *v*ₒ*δ*= 297,72 Hz), 7,85 (AB sys, 4H, *J_{AB}* = 7,99 Hz, *vₒδ*= 226,28 Hz).

### Préparation du composé 3a

Le composé **3a** est préparé selon le schéma réactionnel suivant :

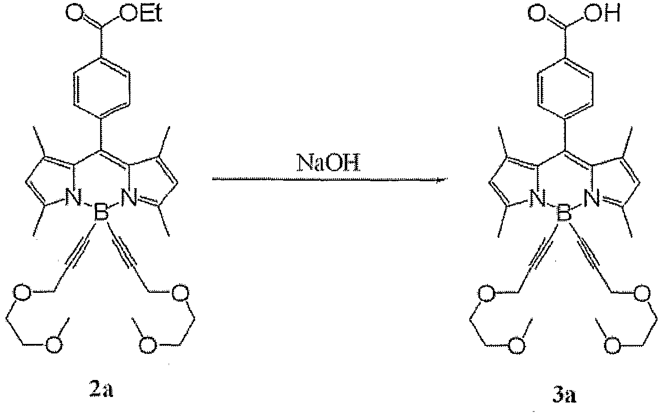

A une solution du composé **2a** (210mg, 0,36 mmol) dans 20 mL d'éthanol a été ajouté 215 mg de soude (5,39 mmol). La solution a été agitée pendant 3 heures à température ambiante. 30-40 mL d'acétate d'éthyle ont été ajoutés. La phase organique a été extraite à l'eau (3x20 mL). Les phases aqueuses ont été rassemblées et acidifiées à l'aide de solution HC11M jusqu'à pH 1-2.

La phase aqueuse a été extraite au dichlorométhane. La phase organique a été séchée sur coton hydrophile puis évaporée à sec pour donner le composé **3a** sous forme d'une poudre orange (190 mg, 95%).

### Caractérisation du composé 3a

UV-Vis (CH₂Cl₂) λ nm (ε, M⁻¹ cm⁻¹) = 501 (72800), 366 (4000), 307 (6400);
UV-Vis (Tampon PBS) λ nm (ε, M⁻¹ cm⁻¹) = 494 (71500), 364 (3900), 307 (4900).

### Préparation du composé 3b

Le composé **3b** est préparé selon le schéma réactionnel suivant :

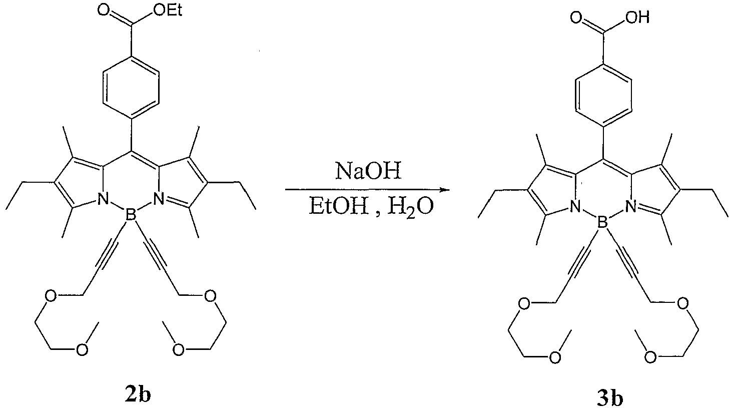

A une solution du composé **2b** (840mg, 1,31 mmol) dans 50 mL d'éthanol ont été ajoutés 2,10 g de soude (0,525 mol). La solution a été agitée pendant une nuit à température ambiante. 30-40 mL d'acétate d'éthyle ont été ajoutés. La phase organique est extraite à l'eau (3x20 mL). Les phases aqueuses ont été rassemblées et acidifiées à l'aide de solution HCl 1M jusqu'à pH 1-2. La phase aqueuse a été extraite au dichlorométhane. La phase organique a été séchée sur coton hydrophile puis évaporée à sec pour donner le composé **3b** sous forme d'une poudre orange (778 mg, 97%). UV-Vis (Tampon PBS) λ nm (ε, M⁻¹ cm⁻¹) = 517 (65000), 378 (3600), 320 (5000);

### Préparation du composé 3c

Le composé **3c** est préparé selon le schéma réactionnel suivant :

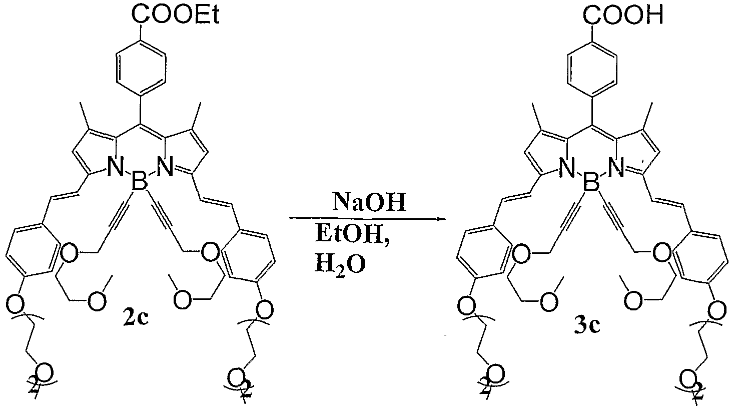

A une solution du composé **2c** (80mg, 0,080 mmol) dans 20 mL d'éthanol ont été ajoutés 130 mg de soude (3,22 mmol). La solution a été agitée pendant une nuit à température ambiante. 10-20 mL d'acétate d'éthyle ont été ajoutés. La phase organique est extraite à l'eau (3x10 mL). Les phases aqueuses ont été rassemblées et acidifiées à l'aide de solution HCl 1M jusqu'à pH 1-2. La phase aqueuse a été extraite au dichlorométhane. La phase organique a été séchée sur coton hydrophile puis évaporée à sec pour donner le composé **3c** sous forme d'une poudre bleue (70 mg, 90%).

### Caractérisation du composé 3c

RMN ¹H (CDCl₃ 300 MHz) : 1,41 (s, 6H), 3,15 (m, 4H), 3,20 (s, 6H), 3,41 (s, 6H), 3,52 (m, 4H), 3,60 (m, 4H), 3,74 (m, 4H), 3,90 (m, 4H), 4,17 (s, 4H), 4,22 (m, 4H), 6,63 (s, 2H), 7,28 (AB sys, 8H, *J_{AB}* = 8,6 Hz, *vₒδ*= 120,59 Hz), 7,61 (AB sys, 4H, *J_{AB}* = 16,12 Hz, *vₒδ*= 191,44 Hz) 7,88 (AB sys, 4H, *J_{AB}* = 8,19 Hz, *v*ₒ*δ*= 144,73 Hz). UV-Vis (CH₂Cl₂) λ nm (ε, M⁻¹ cm⁻¹) = 647 (118000), 373 (67800).

### Préparation du composé 3d

Le composé **3d** est préparé selon le schéma réactionnel suivant :

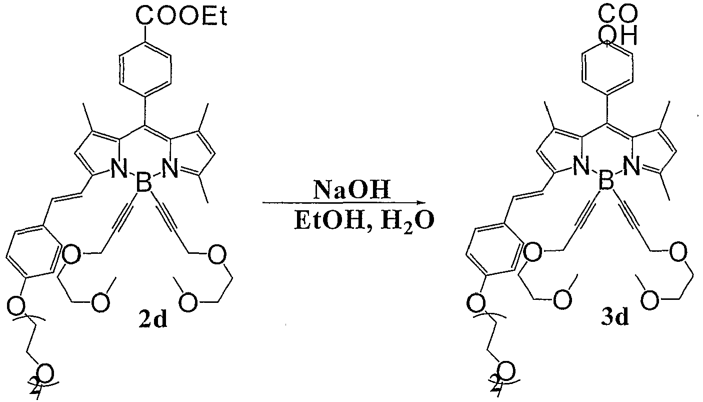

A une solution du composé **2d** (80 mg, 0,10 mmol) dans 20 mL d'éthanol ont été ajoutés 162 mg de soude (4,05 mmol). La solution a été agitée pendant une nuit à température ambiante. 10-20 mL d'acétate d'éthyle ont été ajoutés. La phase organique est extraite à l'eau (3x10 mL). Les phases aqueuses ont été rassemblées et acidifiées à l'aide de solution HCl 1M jusqu'à pH 1-2. La phase aqueuse a été extraite au dichlorométhane. La phase organique a été séchée sur coton hydrophile puis évaporée à sec pour donner le composé **3d** sous forme d'une poudre orange (70 mg, 90%).

### Caractérisation du composé 3d

RMN ¹H (CDCl₃ 300 MHz) :1,36 (s, 3H), 1,39 (s, 3H), 2,74 (s, 3H), 3,26 (s, 6H), 3,32 (m, 4H), 3,40 (s, 3H), 3,56 (m, 6H), 3,73 (m, 2H), 3,88 (m, 2H), 4,18 (m, 6H), 6,03 (s, 1H), 6,59 (s, 1H), 7,22 (AB sys, 4H, *J_{AB}* = 8,67 Hz, *vₒδ*= 180,18 Hz); 7,58 (AB sys, 2H, *J_{AB}* = 16,29 Hz, *vₒδ*= 297,72 Hz), 7,85 (AB sys, 4H, *J_{AB}* = 7,99 Hz, *vₒδ*= 226,28 Hz).
RMN ¹³C (CDCl₃, 300 MHz,) : *δ* = 14,86, 15,08, 16,37, 29,79, 58,91, 59,18, 59,58, 67,63, 68,41, 69,79, 70,88, 71,69, 72,05, 91,43, 128,76, 129,06, 129,69, 130,15, 130,44, 130,86, 134,10, 138,65, 140,39, 140,59, 141,25, 152,61, 155,54, 159,61, 170,40.
UV-Vis (Tampon PBS) λ nm (ε, M⁻¹ cm⁻¹) = 564 (70000), 338 (25000).

### Préparation du composé 4a

Le composé **4a** est préparé selon le schéma réactionnel suivant :

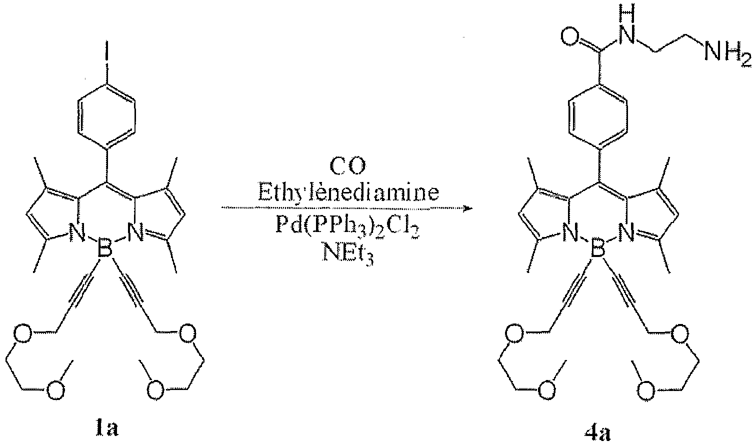

A une solution du composé **la** (200 mg, 0,31 mmol) dans 15 mL de benzène a été ajouté 1 mL d'éthylènediamine (15 mmol), 66 mg de palladium bis-triphénylphosphine bis-chlorure (0,09 mmol) et 1 mL de triéthylamine. La solution a été agitée à 70°C pendant une nuit en faisant "buller" du monoxyde de carbone. Le mélange réactionnel a été extrait au dichlorométhane et lavé à l'eau (3x20 mL). La phase organique a été séchée sur du coton hydrophile et évaporée. Le résidu a été purifié par chromatographie sur colonne de silice (gradient de CH₂Cl₂ 100% à CH₂Cl₂ 75:25) pour donner le composé **4a** sous forme d'une poudre orange (160 mg, 80%).

### Caractérisation du composé 4a

RMN ¹H (CDCl₃, 300 MHz): δ = 1,34 (s, 6H), 2,72 (s, 6H), 3,01 (t, 2H, ³*J* = 5,7 Hz), 3,36 (s, 6H), 3,55 (m, 6H), 3,64 (m, 4H), 4.20 (s, 4H), 6,01 (s, 2H), 6,98 (t, 1H, ³*J* = 5,5 Hz), 7,69 (AB sys, 4H, *J_{AB}* = 8,3 Hz, *vₒδ* = 162,7 Hz);
¹³C {¹H} NMR (CDCl₃, 75,4 MHz,) : δ = 15,0, 16,2, 41,3, 42,2, 59,1, 59,8, 68,7, 71,9, 90,6, 121,9, 127,9, 128,8, 129,4, 134,9, 139,1, 140,4, 141,0, 155,7, 167,0;
RMN ¹¹B (CDCl₃, 128,4 MHz) : δ = -10,3 (s);
UV-Vis (CH₂Cl₂) λ nm (ε, M⁻¹ cm⁻¹) = 500 (64500), 371 (5600)
FAB⁺ m/z : 599,2 ([M+H]⁺, 100) ;
Analyse élémentaire calculée pour C₃₄H₄₃BN₄O₅ : C, 68,23 ; H, 7,24 ; N, 9,36. Trouvé: C, 67,84 ; H, 7,07 ; N, 9,22.

### Préparation du composé 5a

Le composé **5a** est préparé selon le schéma réactionnel suivant :

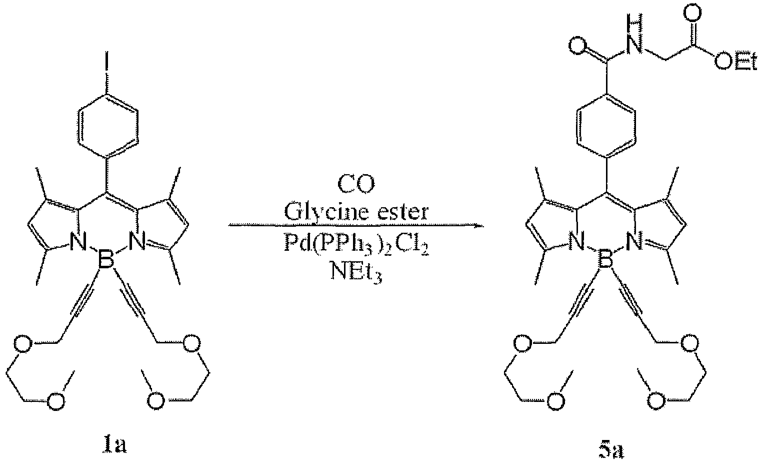

A une solution du composé **la** (100 mg, 0,16 mmol) dans 6 mL de toluène anhydre ont été ajoutés 80 mg de glycine ester d'éthyle (0,47 mmol), 22 mg de palladium bis-triphénylphosphine bis-chlorure (0,03 mmol) et 2 mL de triéthylamine. La solution a été agitée à 80°C pendant 6h en faisant "buller" du monoxyde de carbone. Le mélange réactionnel a été filtré sur Celite et partiellement évaporé. Le résidu a été extrait au dichlorométhane et lavé à l'eau (2x20 mL). La phase organique a été séchée sur du coton hydrophile et évaporée. Le résidu a été purifié par chromatographie sur colonne de gel de silice (AcOEt/Ether de pétrole 50:50) pour donner le composé **5a** sous forme d'une poudre orange (43 mg, 43%).

### Caractérisation du composé 5a

RMN ¹H (CDCl₃, 300 MHz): *δ* = 1,33 (t, 3H, ³*J* = 7,2 Hz), 1,34 (s, 6H), 2,72 (s, 6H), 3,36 (s, 6H), 3,55 (m, 4H), 3,66 (m, 4H), 4,20 (s, 4H), 4,27 (d, 2H, ³*J* = 4,5 Hz), 4,29 (q, 2H, ³*J* = 7,1 Hz), 6,01 (s, 2H), 6,77 (t, 1H, ³*J* = 4,9 Hz), 7,69 (AB sys, 4H, *J_{AB}* = 8,3 Hz, *vₒδ* = 156,4 Hz);
RMN ¹³C {¹H} (CDCl₃, 75,4 MHz,) : *δ* = 14,3, 14,9, 16,2, 42,1, 59,1, 59,8, 61,9, 68,7, 71,9, 91,0, 121,9, 127,9, 129,0, 129,3, 134,2, 139,5, 140,2, 141,0, 155,8, 166,7, 170,2;
RMN ¹¹B (CDCl₃, 128,4 MHz) : δ = -10,3 (s);
UV-Vis (CH₂Cl₂) λ nm (ε, M⁻¹ cm⁻¹) = 501 (84200), 366 (4200), 309 (6500).

### Préparation du composé 6a

Le composé 6a est préparé selon le schéma réactionnel suivant :

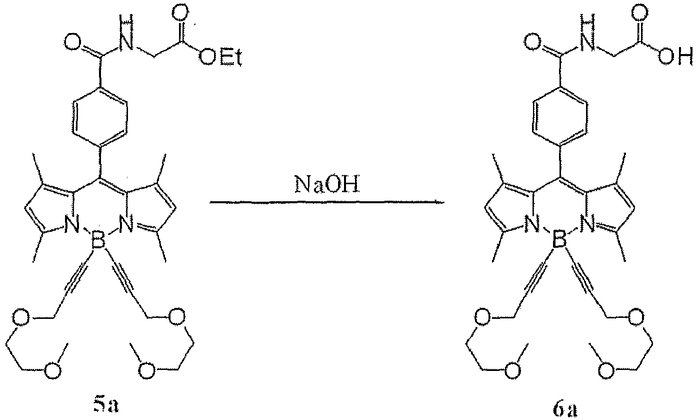

A une solution du composé **5a** (40 mg, 0,06 mmol) dans l'éthanol (10 mL) a été ajoutée une solution aqueuse de soude (60 mg, 1,2 mmol). La solution a été agitée pendant 2h à température ambiante. 10-20 mL d'acétate d'éthyle ont été ajoutés. La phase organique est extraite à l'eau (2x20 mL). Les phases aqueuses ont été rassemblées et acidifiées à l'aide de solution HCl 1M jusqu'à pH 1-2. Le produit a été extrait au dichlorométhane. La phase organique a été séchée sur Na2SO4 puis évaporée à sec pour donner le composé **6a** sous forme d'une poudre orange (35 mg, 90%).

### Caractérisation du composé 6a

RMN ¹H (CDCl₃, 400 MHz) : *δ* = 1,32 (s, 6H), 2,72 (s, 6H), 3,36 (s, 6H), 3,55 (m, 4H), 3,66 (m, 4H), 4,19 (s, 4H), 4,27 (d, 2H, ³*J* = 3,4 Hz), 4,87 (b, 1H), 6,01 (s, 2H), 7,04 (t, 1H, ³*J* = 3,8 Hz), 7,69 (AB sys, 4H, *J_{AB}* = 6,0 Hz, *vₒδ* = 160,2 Hz);
RMNNMR ¹³C {¹H} (CDCl₃, 100 MHz,) : *δ* = 14,9, 16,2, 42,0, 59,0, 59,7, 68,6, 71,8, 90,9, 121,9, 128,0, 129,0, 129,3, 133,8, 139,6, 140,2, 140,9, 155,8, 167,3, 172,3;
RMN ¹¹B (CDCl₃, 128,4 MHz): δ = -10,2 (s);
UV-Vis (CH₂Cl₂) λ nm (ε, M⁻¹ cm⁻¹) = 501 (65000), 366 (3800), 309 (5900);
UV-Vis (Tampon PBS) λ nm (ε, M⁻¹ cm⁻¹) = 496 (59600), 367 (4100), 308 (6100).

### Préparation du composé 7a

Le composé **7a** est préparé selon le schéma réactionnel suivant :

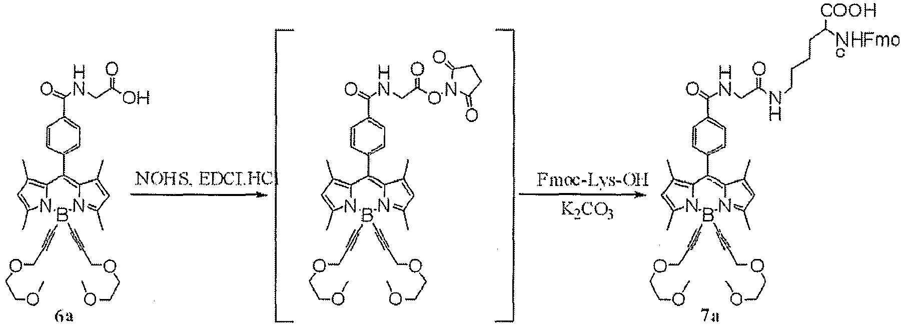

A une solution du composé **6a** (25 mg, 0,04 mmol) et de N-hydroxysuccinimide (7 mg, 0,06 mmol) dans du dichlorométhane (5 mL) ont été ajoutés, à 0°C, 12 mg de N-diméthyle-3-aminopropyle-carbo-diimide (0,06 mmol). Le milieu réactionnel a été agité pendant une nuit en laissant le bain remonter à température ambiante. La solution a été diluée dans du dichlorométhane, lavée avec une solution d'acide chlorhydrique 1M (2 x 20 mL), avec une solution NaHCO₃ 5% puis avec une solution saturée NaCl. La phase organique a été séchée sur Na₂SO₄ puis évaporée à sec sous pression réduite. Le résidu a été dissout dans l'acétonitrile (5 mL). A cette solution a été ajouté une solution de Fmoc-Lys-OH (16 mg, 0,04 mmol) et de K₂CO₃ (11 mg, 0,08 mmol) dans un mélange acétonitrile/eau (5 mL/1 mL). La solution a été agitée à température ambiante pendant 1h, ensuite elle a été extraite à l'acétate d'éthyle. La phase organique a été lavée à l'eau (2 x 20mL), séchée sur MgSO₄ et évaporée. Le résidu a été purifié par chromatographie sur colonne de silice éluée (gradient de AcOEt 100% à AcOEt/EtOH 90:10). Les phases aqueuses ont été rassemblées, acidifiées avec une solution d'acide chlorhydrique 1M jusqu'à pH 1 et extraites au dichlorométhane. La phase organique résultante a été séchée sur MgSO₄ et évaporée. Les différentes fractions ont été rassemblées pour donner le composé **7a** sous forme d'une poudre orange (25 mg, 64%).

### Caractérisation du composé 7a

RMN ¹H (CDCl₃, 300 MHz) : *δ* = 1,27 (s, 6H), 1,35-1,82 (m, 6H), 2,67 (s, 6H), 3,21-3,31 (m, 8H), 3,49 (m, 4H), 3,61 (m, 4H), 3,99-4,36 (m, 10H), 5,95 (s, 2H), 7,22-7,39 (m, 6H), 7,54-7,58 (m, 2H), 7,70 (d, 2H, ³*J* = 7,5 Hz), 7,93 (d, 2H, ³*J* = 7,7 Hz);
RMN ¹³C {¹H} (CDCl₃, 75,4 MHz,) : *δ* = 14,7, 16,0, 23,4, 28,6, 31,5, 31,9, 39,1, 43,3, 53,6, 58,9, 59,6, 67,0, 68,5, 71,7, 90,7, 118,8, 120,0, 121,8, 123,3, 125,1, 127,1, 127,8, 128,0, 129,2, 129,8, 133,9, 139,4, 140,2, 140,9, 141,3, 143,8, 143,9, 155,6, 156,4, 167,5, 169,2;
ESI m/z : 986,4 ([M+Na]⁺, 100).

### Préparation du composé 5a¹³

Le composé **5a**¹³ est préparé selon le schéma réactionnel suivant :

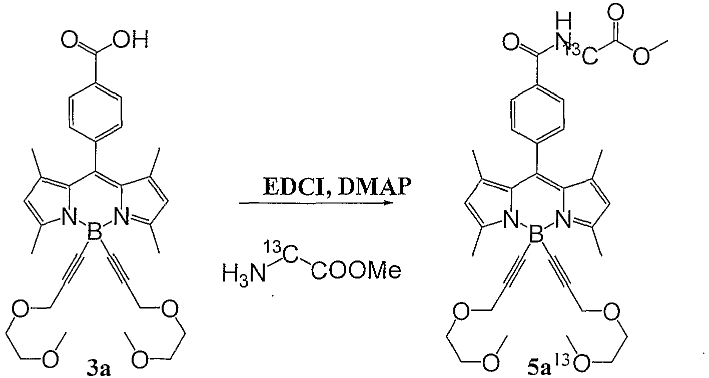

A une solution du composé **3a** (570 mg, 1,025 mmol) dans du dichlorométhane (30 mL) a été ajoutés, à 0°C, 294 mg de N-diméthyle-3-aminopropyle-carbo-diimide (1,537 mmol) et 187 mg de diméthyle-amino-pyridine (1,537 mmol). Le milieu réactionnel a été agité pendant une heure à température ambiante. A cette solution a été ensuite ajoute 142 mg de glycine ester marqué au carbone 13 (1,127 mmol). Le milieu réactionnel a été agité pendant une heure à température ambiante. La solution a été diluée dans du dichlorométhane, lavée a l'eau (2 x 20 mL), avec une solution NaHCO₃ 5% puis avec une solution saturée NaCl. La phase organique a été séchée sur Na₂SO₄ puis évaporée à sec sous pression réduite. Le résidu a été purifié par chromatographie sur colonne de silice éluée avec un gradient acétate d'éthyle/éther de pétrole 20/80, 30/70, 40/60, pour donner le compose **5a**¹³ sous forme de poudre orange (527 mg, 82%)

### Caractérisation du composé 5a¹³

RMN ¹H (CDCl₃ 300 MHz) : 1,32 (s, 6H), 2,71 (s, 6H), 3,34 (s, 6H), 3,52 (m, 4H), 3,64 (m, 4H), 4,02 (d, 1H, ¹*J* = 141,3 Hz, ³*J* = 4,4 Hz), 4,18 (s, 4H), 4,50 (d, 1H, ¹*J* = 141,3 Hz, ³*J* = 4,4 Hz), 6,00 (s, 2H), 6,85 (s, 1H), 7,68 (AB sys, 4H, *J_{AB}* = 8 Hz, *vₒδ*= 159,56 Hz).
RMN ¹³C (CDCl₃, 300 MHz,): *δ* = 14,72, 16, 41,72, 52,48, 58,86, 59,54, 68,44, 71,66, 76,57, 77, 77,42, 121,66, 127,78, 128,71, 129,06, 133,88, 139,24, 140, 140,72, 155,51, 166,58, 169,976, 170,79.

### Préparation du composé 6a¹³

Le composé **6a**¹³ est préparé selon le schéma réactionnel suivant :

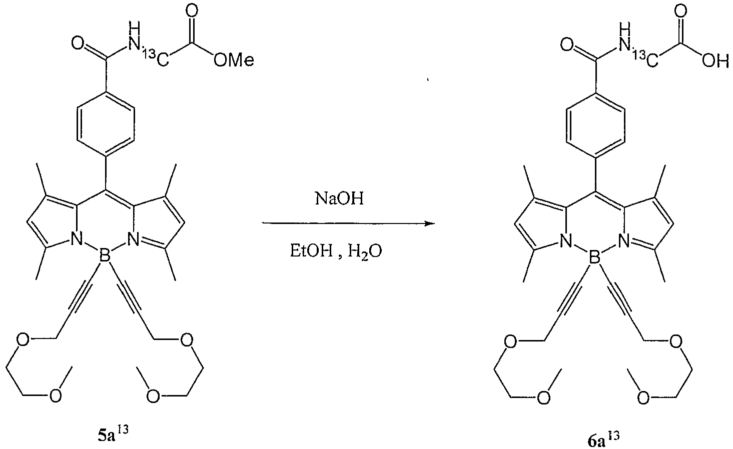

A une solution du composé **5a**¹³ (500 mg, 0,795 mmol) dans l'éthanol (30 mL) a été ajoutée une solution aqueuse de soude (1,27 g, 32 mmol). La solution a été agitée pendant 2h à température ambiante. 20-30 mL d'acétate d'éthyle ont été ajoutés. La phase organique est extraite à l'eau (3x20 mL). Les phases aqueuses ont été rassemblées et acidifiées à l'aide de solution HCl 1M jusqu'à pH 1-2. La phase aqueuse a été extraite au dichlorométhane. La phase organique a été séchée sur coton hydrophile puis évaporée à sec pour donner le composé **6a**¹³ sous forme d'une poudre orange (464 mg, 95%).

### Caractérisation du composé 6a¹³

RMN ¹H (CDCl₃ 300 MHz) : *δ* = 1,29 (s, 6H), 2,70 (s, 6H), 3,34 (s, 6H), 3,52 (m, 4H), 3,65 (m, 4H), 3,93 (s, 1H, ¹*J* = 140,6 Hz), 4,17 (s, 4H), 4,39 (s, 1H, ¹*J* = 140,6 Hz), 7,67 (AB sys, H, *J_{AB}* = 7,72 Hz, *vₒδ*= 177,95 Hz).
¹³C RMN (CDCl₃, 300 MHz,) : *δ* =14,72, 15,43, 16,03, 42,20, 43,02, 54,94, 58,36, 58,85, 59,55, 68,46, 71,63, 76,57, 90,76, 121,74, 127,94, 128,74, 129,05, 133,70, 139,35, 139,94, 140,68, 140,78, 155,26, 155,48, 155,57, 167,35.

### Préparation du composé 7a¹³

Le composé **7a¹³** est préparé selon le schéma réactionnel suivant :

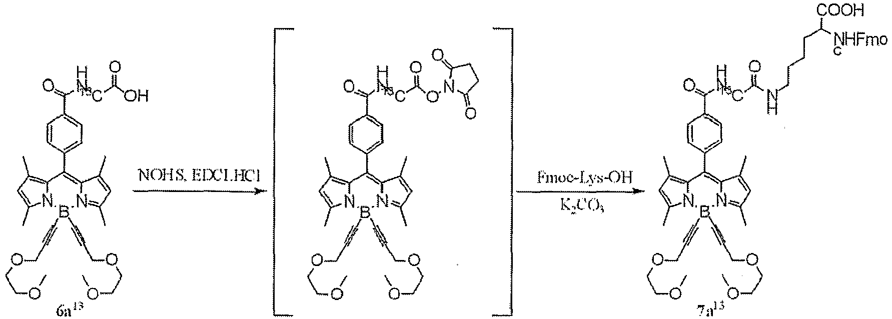

A une solution du composé **6a¹³** (430 mg, 0,7 mmol) et de N-hydroxysuccinimide (120 mg, 1,05 mmol) dans du dichlorométhane (50 mL) ont été ajoutés, à 0°C, 207 mg de N-diméthyle-3-aminopropyle-carbo-diimide (1,05 mmol). Le milieu réactionnel a été agité pendant une nuit en laissant le bain remonter à température ambiante. La solution a été diluée dans du dichlorométhane, lavée avec une solution d'acide chlorhydrique 1M (3 x 20 mL), avec une solution NaHCO₃ 5% puis avec une solution saturée NaCl. La phase organique a été séchée sur Na₂SO₄ puis évaporée à sec sous pression réduite. Le résidu a été dissout dans l'acétonitrile (50 mL). A cette solution, a été ajouté une solution de Fmoc-Lys-OH (566 mg, 1,40 mmol) et de K₂CO₃ (193 mg, 1,40 mmol) dans un mélange acétonitrile/eau (15 mL/5 mL). La solution a été agitée à température ambiante pendant 2h, ensuite elle a été extraite à l'acétate d'éthyle. La phase organique a été lavée à l'eau (3 x 20mL), séchée sur MgSO₄ et évaporée. Le résidu a été purifié par chromatographie sur colonne de silice éluée avec un gradient allant de AcOEt 100% à AcOEt/EtOH 90:10. Les différentes fractions ont été rassemblées pour donner le composé **7a¹³** sous forme d'une poudre orange (539 mg, 80%).

### Caractérisation du composé 7a¹³

RMN ¹H (MeOD 400 MHz 60°C) : *δ* =1,18 (m, 1H), 1,22 (s, 6H), 1,40 (m, 2H), 1,53 (m, 2H), 2,71 (s, 6H), 3,23 (t, 2H, ³*J* = 6,72 Hz) 3,34 (s, 6H), 3,51 (m, 4H), 3,63 (m, 4H), 4,14 (s, 4H), 4,18 (t, 1H, ³*J* = 5,10 Hz),4,21 (d, 2H, ¹JHC¹³= 140Hz), 6,04 (s, 2H), 7,25 (m, 2H), 7,33 (m, 4H),7,61 (m, 2H), 7,71 (d, 2H), 8,01 (d, 2H).

### Préparation du composé 5b

Le composé **5b** est préparé selon le schéma réactionnel suivant :

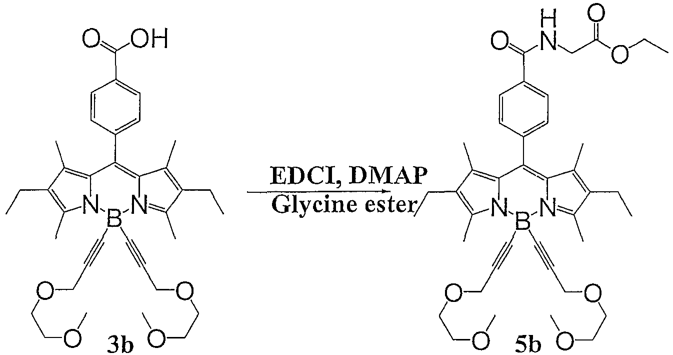

A une solution du composé **3b** (600 mg, 0,979 mmol) dans du dichlorométhane (50 mL) ont été ajouté, à 0°C, 280 mg de N-diméthyle-3-aminopropyle-carbo-diimide (1,47 mmol) et 180 mg de diméthyle-amino-pyridine (1,47 mmol). Le milieu réactionnel a été agité pendant une heure à température ambiante. A cette solution a été ensuite ajoute 205 mg de glycine ester (1,47 mmol). Le milieu réactionnel a été agité pendant une heure à température ambiante. La solution a été diluée dans du dichlorométhane, lavée à l'eau (2 x 20 mL), avec une solution NaHCO₃ 5% puis avec une solution saturée NaCl. La phase organique a été séchée sur Na₂SO₄ puis évaporée à sec sous pression réduite. Le résidu a été purifié par chromatographie sur colonne de silice éluée avec un gradient acétate d'éthyle/éther de pétrole 20/80, 30/70, 40/60, pour donner le compose **5b** sous forme de poudre orange (560 mg, 82%).

### Caractérisation du composé 5b

RMN ¹H (CDCl₃ 300 MHz): 0,97 (t, 6H, ³*J* = 7,35 Hz), 1,22 (s, 6H) 1,31 (t, 2H, ³*J* = 7,10 Hz), 2,28 (q, 4H, ³*J* = 7,5 Hz) 2,68 (s, 6H), 3,34 (s, 6H), 3,53 (m, 4H), 3,64 (m, 4H), 4,18 (s, 4H), 4,25 (m, 4H), 6,84 (t, 1H, ³*J* = 4,7 Hz), 7,78 (AB sys, 4H, *J_{AB}* = 8,5 Hz, *vₒδ*= 300,4 Hz);
RMN ¹³C (CDCl₃, 300 MHz,) : *δ*= 12,08, 14,05, 14,25, 14,75, 15,34, 17,38, 29,72, 42,05, 59, 59,76, 61,85, 68,55, 71,83, 90,62, 127,81, 128,66, 129,16, 133,11, 133,91, 136,02, 138,60, 140,27, 154,04, 166,80, 170,14.

### Préparation du composé 6b

Le composé **6b** est préparé selon le schéma réactionnel suivant :

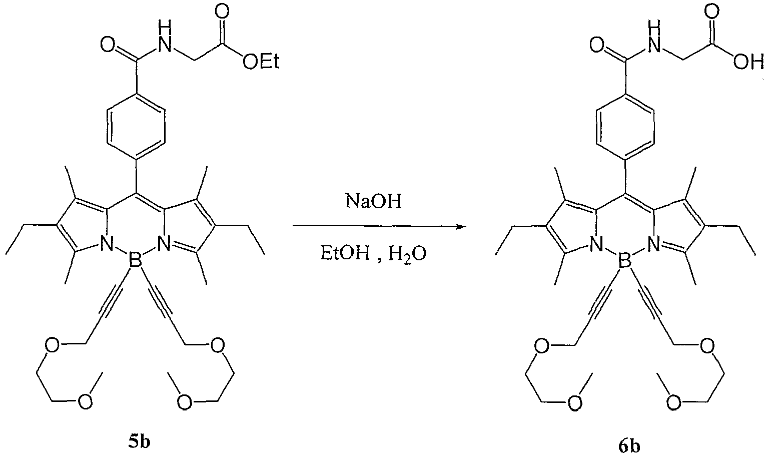

A une solution du composé **5b** (510 mg, 0,732 mmol) dans l'éthanol (50 mL) a été ajoutée une solution aqueuse de soude (1,20 g, 30 mmol). La solution a été agitée pendant 2h à température ambiante. 20-30 mL d'acétate d'éthyle ont été ajoutés. La phase organique est extraite à l'eau (3x20 mL). Les phases aqueuses ont été rassemblées et acidifiées à l'aide de solution HCl 1M jusqu'à pH 1-2. La phase aqueuse a été extraite au dichlorométhane. La phase organique a été séchée sur coton hydrophile puis évaporée à sec pour donner le composé **6b** sous forme d'une poudre orange (490 mg, quantitatif).

### Caractérisation du composé 6b

RMN ¹H (CDCl₃ 300 MHz) : *δ* =0,96 (t, 6H, ³*J*= 7,3 Hz), 1,22 (s, 6H), 2,30 (q, 4H, ³*J* = 7,3 Hz), 2,68 (s, 6H), 3,34 (s, 6H), 4,19 (s, 4H), 3,54 (m, 4H), 3,66 (m, 4H), 4,18 (s, 4H), 4,26 (d, 2H, ³*J* = 4,8 Hz), 7,13 (t, 1H, ³*J* = 4,8 Hz), 7,40 (AB sys, H, *J_{AB}* = 6,0 Hz, *v*ₒ*δ*= 160,2 Hz);
RMN ¹³C (CDCl₃, 300 MHz,) : *δ* =12,08, 14,03, 14,73, 17,35, 41,93, 58,91, 59,71, 68,48, 71,74, 90,51, 127,90, 128,60, 129,18, 133,14, 133,57, 136,01, 138,54, 140,39, 154,04, 167,35, 172,29.

### Préparation du composé 7b

Le composé **7b** est préparé selon le schéma réactionnel suivant :

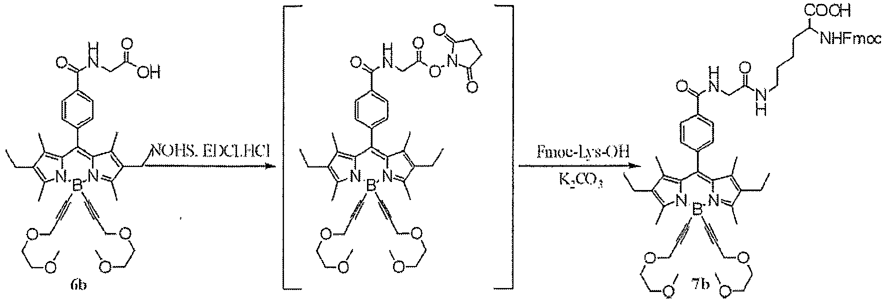

A une solution du composé **6b** (430 mg, 0,643 mmol) et de N-hydroxysuccinimide (112 mg, 0,968 mmol) dans du dichlorométhane (50 mL) ont été ajoutés, à 0°C, 190 mg de N-diméthyle-3-aminopropyle-carbo-diimide (0,968 mmol). Le milieu réactionnel a été agité pendant une nuit en laissant le bain remonter à température ambiante. La solution a été diluée dans du dichlorométhane, lavée avec une solution d'acide chlorhydrique 1M (3 x 20 mL), avec une solution NaHCO₃ 5% puis avec une solution saturée NaCl. La phase organique a été séchée sur Na₂SO₄ puis évaporée à sec sous pression réduite. Le résidu a été dissout dans l'acétonitrile (50 mL). A cette solution a été ajouté une solution de Fmoc-Lys-OH (522 mg, 1,29 mmol) et de K₂CO₃ (180 mg, 1,29 mmol) dans un mélange acétonitrile/eau (15 mL/5 mL). La solution a été agitée à température ambiante pendant 2h, ensuite elle a été extraite à l'acétate d'éthyle. La phase organique a été lavée à l'eau (3 x 20mL), séchée sur MgSO₄ et évaporée. Le résidu a été purifié par chromatographie sur colonne de silice éluée avec un gradient de AcOEt 100% à AcOEt/EtOH 90:10. Les différentes fractions ont été rassemblées pour donner le composé **7b** sous forme d'une poudre orange (491 mg, 75%).

### Caractérisation du composé 7b

NMR ¹H (MeOD 400 MHz 60°C) : *δ* =0,98 (t, 6H, ³*J* = 7,50 Hz), 1,26 (s, 6H), 1,41 (m, 2H), 1,55 (m, 2H), 2,33 (q, 4H, ³*J* = 7,50 Hz), 2,71 (s, 6H), 3,24 (t, 2H,³*J* = 5,10 Hz) 3,34 (s, 6H), 3,51 (m, 4H), 3,63 (m, 4H), 4,06 (s, 3H), 4,14 (s, 4H), 4,19 (t, 1H, ³*J* = 5,10 Hz), 4,37 (m, 2H), 7,30 (m, 6H), 7,62 (m, 2H), 7,87 (AB sys, H, *J_{AB}* = 7,66 Hz, *v*ₒ*δ*= 112,84 Hz).

### Exemple 2 - Synthèse d'un peptide comportant une lysine marquée par un composé de formule générale (I) (composé de formule générale (II) selon l'invention)

Le composé **7a** (lysine marquée) préparé selon l'exemple 1 est utilisé dans un synthétiseur automatique de peptide et substitué à la lysine de la position 16 de la séquence d'acides aminés suivante correspondant au peptide bêta-amyloïde 1-42 humain :
H-Asp-Ala-Glu-Phe-Arg-His-Asp-Ser-Gly-Tyr-Glu-Val-His-His-Gln-**Lys***-Leu-Val-Phe-Phe-Ala-Glu-Asp-Val-Gly-Ser-Asn-Lys-Gly-Ala-Ile-Ile-Gly-Leu-Met-Val-Gly-Gly-Val-Val-Ile-Ala-OH (SEQ ID NO:1).

Le peptide est utilisé dans les exemples 3, 4 et 5 et est appelé par la suite peptide amyloïde marqué.

### Protocole commun aux exemples 3 et 4

Des cellules PC12 (issues d'une tumeur de glande médullo-surrénale de rat mâle) sont ensemencées dans des boîtes de Pétri à fond de verre préalablement traitées au collagène et à la polyornithine. Elles sont cultivées pendant 7 jours dans une atmosphère à 5% de CO₂ à 37 °C dans du milieu RPMI 1640 contenant de la glutamine et supplémenté par 10% de sérum de cheval, 5% de sérum de veau foetal, 50 U/mL de pénicilline et 50 µg/mL de streptomycine. Pour induire la différenciation des cellules, on ajoute au milieu, 24 heures après l'ensemencement, 50 ng/mL de facteur de croissance neurotrophile (NGF) 2.5S murin.

Le jour précédant la mesure, le peptide bêta-amyloïde 1-42 non marqué de SEQ. ID. NO:1 ou le peptide inverse correspondant (42-1) non marqué de SEQ ID NO:2 est ajouté à diverses concentrations comprises entre 10 et 1000 nmol.L⁻¹.

Le jour suivant, cette préparation est lavée 3 fois par du milieu de Krebs à température ambiante et placée sur un microscope inversé couplé à un spectrofluorimètre. Un spectre de fluorescence (excitation 480 nm ; émission 495-600 nm) de la ligne de base est enregistré avant l'addition du peptide amyloïde marqué (250 nmol.L⁻¹). Un second spectre de fluorescence est enregistré après 10 minutes d'incubation.

### Exemple 3 - mesure de la valeur de l'indice spectral du composé 7a couplé au peptide bêta-amyloïde 1-42 après préincubation du peptide bêta-amyloïde non marqué

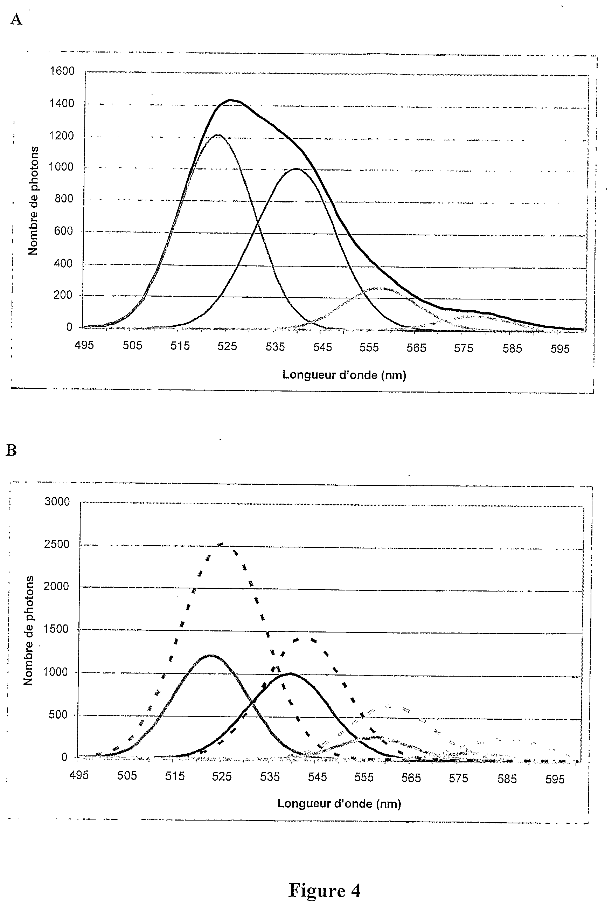

Le spectre de la ligne de base est soustrait du spectre de fluorescence du peptide amyloïde marqué. Le spectre obtenu est décomposé en 4 courbes gaussiennes élémentaires par déconvolution et régression non-linéaire comme illustré à la **Figure 4****,** ce spectre de fluorescence (tracé noir) peut être décomposé en courbes gaussiennes élémentaires (tracés en différents niveaux de gris) à l'aide d'un logiciel de déconvolution de spectres (Peakfit, Seasolve Software Inc., www.seasolve.com) (**Graphe A**). Le rapport de l'amplitude (mesurée graphiquement ou bien par le logiciel de déconvolution) des courbes gaussiennes centrées respectivement à 525 nm et 540 nm définit un indice spectral décrivant l'étalement du spectre.

Le **Graphe B** représente la déconvolution du spectre de fluorescence du peptide amyloïde marqué enregistré en présence de cellules PC12 avant préincubation (tracés continus) et après préincubation (tracés en pointillés) des cellules en présence de peptide bêta-amyloïde 1-42 non marqué. On constate une augmentation importante de l'amplitude de la courbe gaussienne centrée à 525 nm qui se traduit par une augmentation de l'indice spectral lorsque les cellules ont été placées préalablement en présence de peptide bêta-amyloïde 1-42 non marqué.

On observe ainsi que la préincubation des cellules avec le peptide bêta-amyloïde 1-42 non marqué conduit à une modification du spectre et la présence du peptide bêta-amyloïde 1-42 peut être détectée dans un échantillon.

### Exemple 4 - mesure de la valeur de l'indice spectral du composé 7a couplé au peptide bêta-amyloïde 1-42 après préincubation de différentes concentrations de peptide bêta-amyloïde non marqué

Selon le même principe expérimental, la valeur de l'indice spectral du composé **7a** couplé au peptide bêta-amyloïde 1-42 est mesurée après préincubation de différentes concentrations de peptide bêta-amyloïde non marqué de SEQ ID NO:1.

Les cellules PC12 sont préincubées 12 heures en présence de peptide bêta-amyloïde 1-42 ou 42-1 non marqué puis rincées avant d'être placées sous un microscope couplé à un spectrofluorimètre. Un premier spectre (ligne de base) est enregistré.

On ajoute ensuite le peptide bêta-amyloïde marqué. Un spectre est enregistré 10 minutes après l'addition du composé fluorescent.

L'indice spectral est le rapport entre l'amplitude de la première et celle de la deuxième gaussienne calculée par déconvolution (voir la **Figure 4**).

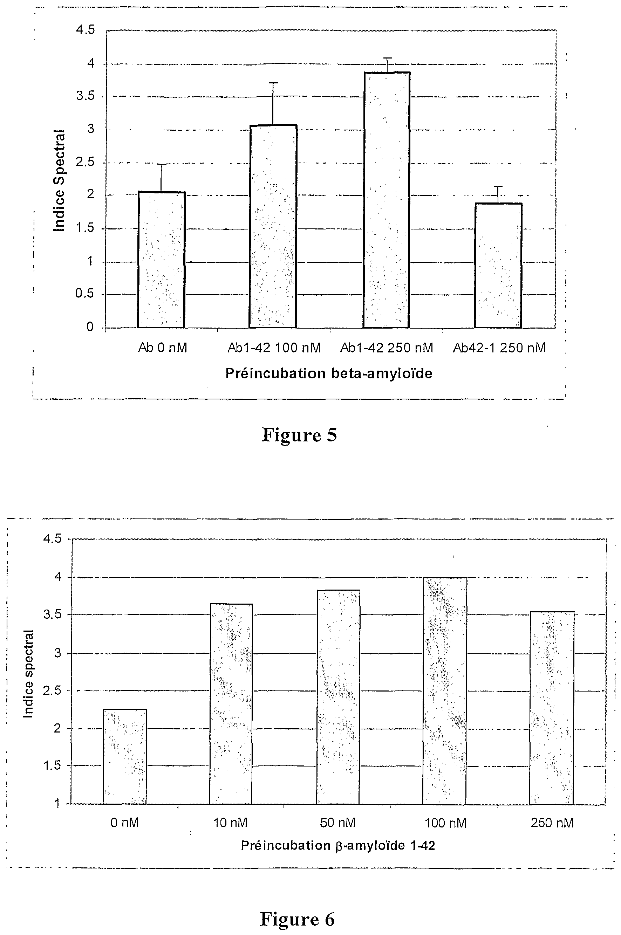

Les résultats sont représentés à la **Figure 5** **:**
- En l'absence de peptide bêta-amyloïde 1-42 non marqué (premier histogramme) ;
- En présence de 100 nM de peptide bêta-amyloïde 1-42 non marqué (second histogramme) ;
- En présence de 250 nM de peptide bêta-amyloïde 1-42 non marqué (troisième histogramme) ;
- En présence de 250 nM de peptide bêta-amyloïde 42-1 non marqué (quatrième histogramme).

La valeur de l'indice spectral de fluorescence de la sonde varie en fonction de la concentration de peptide bêta-amyloïde 1-42 non marqué présent pendant la phase de préincubation des cellules. On constate également une absence de modification de la valeur de l'indice spectral, lorsque les cellules sont incubées en présence de peptide b-amyloïde 42-1 inactif. (Student t-test : *** p<0.5% par rapport à Ab 0 nM; °° p<1% par rapport à Ab 1-42 250 nM). Ce graphe est représentatif de 4 expériences indépendantes mesurées en triplicate.

La **Figure 5** montre que la variation de la valeur du rapport est spécifiquement induite par le peptide bêta-amyloïde 1-42 et que cette valeur augmente avec l'augmentation de concentration en peptide préincubé ; en outre, on observe que le peptide bêta-amyloïde 42-1 n'a aucun effet significatif sur la valeur du rapport.

Ainsi, l'analyse du spectre de fluorescence de la sonde permet d'évaluer la concentration de peptide bêta-amyloïde à laquelle les cellules ont été pré-incubées.

### Exemple 5 - Variation de la valeur de l'indice spectral du peptide bêta-amyloïde 1-42 couplé au composé 7a en présence d'érythrocytes de rat préincubés avec le peptide bêta-amyloïde 1-42 non marqué

Un échantillon de sang de rat (1 à 3 ml) est recueilli dans un tube hépariné. L'échantillon est dilué avec du milieu de Krebs puis centrifugé trois fois en supprimant la couche tampon (« buffy coat »). Les érythrocytes obtenus sont ensuite incubés à 37°C pendant 2 heures en présence du peptide bêta-amyloïde 1-42 non marqué ou le peptide inverse correspondant (42-1) non marqué à diverses concentrations (10-1000 nmol.L⁻¹). Après un lavage supplémentaire, les cellules sont placées dans un spectrofluorimètre et un spectre de fluorescence (excitation 480 nm ; émission 495-600 nm) de la ligne de base est enregistré avant l'addition du peptide amyloïde marqué (250 nmol.L⁻¹). Un second spectre de fluorescence est enregistré après 10 minutes d'incubation en présence du peptide amyloïde marqué.

Le spectre de la ligne de base est soustrait du spectre de fluorescence du peptide bêta-amyloïde marqué. Le spectre obtenu est décomposé en 4 courbes gaussiennes élémentaires par déconvolution et régression non-linéaire. Le rapport de l'amplitude de la gaussienne centrée autour de 510 nm sur l'amplitude de la gaussienne centrée autour de 530 nm est calculé. Sa valeur varie en fonction de la concentration de peptide bêta-amyloïde 1-42 non marqué en présence de laquelle les cellules ont été incubées comme illustré dans la **Figure 6****.**

L'histogramme de la **Figure 6** montre que la valeur de l'indice spectral augmente en fonction de la concentration de peptide bêta-amyloïde non marqué utilisée pendant la préincubation des cellules.

## Revendications

1. Procédé de préparation de composé de formule générale (I) 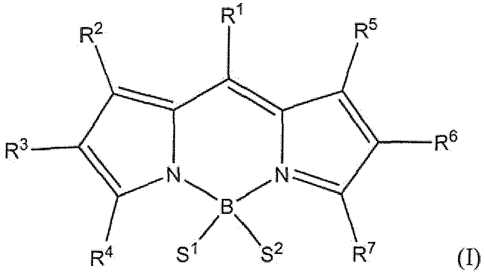 dans laquelle :
- **R**¹ est choisi dans le groupe constitué par -Ar-CO-Z ; un atome d'hydrogène (-H) ; -L-H ; -G et -L-G ;
où Ar, Z, L et G sont définis ci-après ;
- **R³, R⁴, R⁶** et **R⁷** sont choisis indépendamment les uns des autres dans le groupe constitué par -(Ar)ₘ-CO-Z ; un atome d'hydrogène (-H) ; -L-H ; -G et -L-G ;
où m est 0 ou 1 ;
étant entendu qu'un seul des substituants **R¹, R³, R⁴, R⁶** et **R⁷** est -Ar-CO-Z ou - (Ar)ₘ-CO-Z ;
- **R²** et **R⁵** identiques ou différents sont choisis indépendamment l'un de l'autre dans le groupe constitué par un atome d'hydrogène (-H) ; -L-H ; -G et -L-G ;
où L et G sont définis ci-après ;
- **S¹** et **S²** sont des groupements hydrophiles, identiques ou différents, de formule -C≡C-L'-A ;
dans laquelle L' et A sont définis ci-après ;
- **Ar** est choisi parmi un arylène ou un hétéroarylène en C₅-C₁₄ sur lequel le groupe -CO-Z est en position ortho, méta ou para, de préférence para ;
- **Z** est choisi parmi un groupement -OH, -O-succinimide, -O-maléimide, -N-glycine, -N-lysine, -Y-L"-NH₂, -Y-L"-COOH, -Y-L"-SH où **Y** est choisi parmi les atomes de N et O et **L"** est défini ci-après ;
- **L** et **L"** sont choisis indépendamment l'un de l'autre parmi une liaison simple ; une chaîne carbonée saturée éventuellement ramifiée en C₁-C₁₀, de préférence en C₁-C₆ ; un arylène en C₆-C₁₆ sur lequel les groupements -H ou -G pour L et -NH₂, -COOH ou -SH pour L" sont en position ortho, méta ou para, de préférence para ; un alkénylène en C₂-C₄ ; un alkynylène en C₂-C₄ ; une chaîne carbonée linéaire ou ramifiée en C₁-C₂₀ interrompue par 1 à 10 atomes d'oxygène ; une chaîne carbonée saturée, linéaire ou ramifiée en C₁-C₂₀ interrompue par une à quatre fonctions amides -CO-NH- ; un segment nucléotidique et/ou un segment de sucres ;
- **G** est choisi dans le groupe constitué par l'ester succinimidyle, l'ester sulfosuccinimidyle, l'isothiocyanate, l'isocyanate, l'iodoacétamide, la maléimide, les halosulfonyles, les phosphoramidites, les alkylimidates en C₂-C₅, les arylimidates en C₆-C₁₀, les halogénoacides, de préférence, les chloroacides et les bromoacides, les hydrazines substituées par un alkyle en C₁-C₄. les hydroxylamines substituées par un alkyle en C₁-C₄, les carbodiimides et les perfluorophénols ;
- **L'** est choisi parmi une liaison simple ; un alkénylène en C₁-C₁₀ ou une chaîne carbonée saturée, linéaire ou ramifiée en C₁-C₂₀ interrompue par 1 à 10 atomes d'oxygène ;
- **A** représente un groupement choisi parmi un alkyle en C₁-C₄, de préférence, le méthyle ; un groupement phosphate ou un groupement sulfonate,
ledit procédé étant **caractérisé en ce qu'**il comprend la transformation de l'intermédiaire de synthèse **P-(Ar)ₘ-Q** en composé de formule générale (I) par réaction avec une source de carbone en présence d'un nucléophile choisi parmi l'eau, un alcool, une amine ou un thiol et d'un catalyseur au palladium ;
ledit intermédiaire est tel que **P** est un groupement de structure identique au composé de formule générale (I) à préparer à l'exception du radical R¹ ou R³ ou R⁴ ou R⁶ ou R⁷ selon celui qui est le groupement -Ar-CO-Z pour R¹ ou -(Ar)ₘ-CO-Z pour R³ ou R⁴ ou R⁶ ou R⁷, ledit radical étant une liaison fixée à -(Ar)ₘ-Q ; Ar est tel que défini pour la formule (I), **m** est 0 ou 1, étant entendu que m est 1 si R¹ est la fonction carbonyle -Ar-CO-Z et **Q** est choisi parmi un atome d'halogène ; un -O-triflate ; un - O-tosylate ou un -O-mésylate.

2. Procédé selon la revendication 1, **caractérisé en ce que** R² et R⁵ sont identiques, R³ et R⁶ sont identiques, R⁴ et R⁷ sont identiques et S¹ et S² sont identiques.

3. Procédé selon l'une quelconque des revendications précédentes, **caractérisé en ce que** Ar est choisi parmi le benzène, le naphtalène, l'anthracène, le pyrène, la pyridine, la pyrimidine, le thiophène ou le pyrrole.

4. Procédé selon l'une quelconque des revendications précédentes, **caractérisé en ce que** A est choisi parmi un méthyle, un propyl sulfonate, un éthyl sulfonate ou un méthylphosphate.

5. Procédé selon l'une quelconque des revendications précédentes, **caractérisé en ce que** la chaîne carbonée saturée, linéaire ou ramifiée en C₁-C₂₀ interrompue par 1 à 10 atomes d'oxygène est un poly(oxyde d'éthylène) ou un poly(oxyde de propylène) dont le motif se répète entre une et six fois.

6. Molécule biologique marquée de formule générale (II) :
P-(Ar)ₘ -CO-(X)ₙ-T (II)
dans laquelle:
- **P** est le composé de formule générale (I)
dans laquelle :
- R¹ est choisi dans le groupe constitué par -Ar-CO-Z ; un atome d'hydrogène (-H) ; -L-H ; -G et -L-G ;
où Ar, Z, L et G sont définis ci-après ;
- R³, R⁴, R⁶ et R⁷ sont choisis indépendamment les uns des autres dans le groupe constitué par (Ar)ₘ-CO-Z ; un atome d'hydrogène (-H) ; -L-H ; -G et -L-G ;
où m est 0 ou 1 ;
étant entendu qu'un seul des substituants R¹, R³, R⁴, R⁶ et R⁷ est -Ar-CO-Z ou - (Ar)ₘ-CO-Z ;
- R² et R⁵ identiques ou différents sont choisis indépendamment l'un de l'autre dans le groupe constitué par un atome d'hydrogène (-H) ; -L-H ; -G et -L-G ;
où L et G sont définis ci-après ;
- S¹ et S² sont des groupements hydrophiles, identiques ou différents, de formule -C=C-L'-A ;
dans laquelle L' et A sont définis ci-après ;
- Ar est choisi parmi un arylène ou un hétéroarylène en C₅-C₁₄ sur lequel le groupe -CO-Z est en position ortho, méta ou para, de préférence para ;
- Z est choisi parmi un groupement -OH, -O-succinimide, -O-maléimide, -N-glycine, -N-lysine, -Y-L"-NH₂, -Y-L"-COOH, -Y-L"-SH où Y est choisi parmi les atomes de N et O et L" est défini ci-après ;
- L et L" sont choisis indépendamment l'un de l'autre parmi une liaison simple ; une chaîne carbonée saturée éventuellement ramifiée en C₁-C₁₀, de préférence en C₁-C₆ ; un arylène en C₆-C₁₆ sur lequel les groupements -H ou -G pour L et -NH₂, -COOH ou -SH pour L" sont en position ortho, méta ou para, de préférence para ; un alkénylène en C₂-C₄ ; un alkynylène en C₂-C₄ ; une chaîne carbonée linéaire ou ramifiée en C₁-C₂₀ interrompue par 1 à 10 atomes d'oxygène ; une chaîne carbonée saturée, linéaire ou ramifiée en C₁-C₂₀ interrompue par une à quatre fonctions amides -CO-NH- ; un segment nucléotidique et/ou un segment de sucres ;
- G est choisi dans le groupe constitué par l'ester succinimidyle, l'ester sulfosuccinimidyle, l'isothiocyanate, l'isocyanate, l'iodoacétamide, la maléimide, les halosulfonyles, les phosphoramidites, les alkylimidates en C₂-C₅, les arylimidates en C₆-C₁₀, les halogénoacides, de préférence, les chloroacides et les bromoacides, les hydrazines substituées par un alkyle en C₁-C₄, les hydroxylamines substituées par un alkyle en C₁-C₄, les carbodiimides et les perfluorophénols ;
- L' est choisi parmi une liaison simple ; un alkénylène en C₁-C₁₀ ou une chaîne carbonée saturée, linéaire ou ramifiée en C₁-C₂₀ interrompue par 1 à 10 atomes d'oxygène ;
- A représente un groupement choisi parmi un alkyle en C₁-C₄, de préférence, le méthyle ; un groupement phosphate ou un groupement sulfonate à l'exception du radical R¹ ou R³ ou R⁴ ou R⁶ ou R⁷ qui est le groupement -(Ar)ₘ-CO-Z dans la formule générale (I), ledit radical est alors une liaison à -(Ar)ₘ -CO-(X)ₙ-T ;
- **Ar** est choisi parmi un arylène ou un hétéroarylène en C₅-C₁₀ sur lequel le groupe-CO-Z est en position ortho, méta ou para, de préférence para ;
- **m** est 0 ou 1 étant entendu que m est 1 si le groupement -(Ar)ₘ-CO-(X)ₙ-T est substitué à R¹ ;
- **X** est un espaceur porteur d'une fonction carboxylique, amine ou thiol ;
- **n** est un nombre entier égal à 0 ou 1 ;
- **T** est une molécule biologique.

7. Molécule biologique marquée selon la revendication 6, **caractérisée en ce que** T est choisi parmi un acide aminé naturel ou synthétique, un polypeptide, la biotine ou un de ses dérivés ou analogues structuraux, un nucléotide ou un acide nucléique.

8. Molécule biologique marquée selon la revendication 6 ou 7, **caractérisée** en que T est un peptide dérivé du peptide bêta-amyloïde 1-42 de SEQ ID NO:1,

9. Molécule biologique marquée selon l'une quelconque des revendications 6 à 8, **caractérisée en ce que** X est choisi parmi une chaîne comprenant au moins un acide aminé ou un alkylène en C₁-C₆ pouvant être interrompu par 2 ou 3 atomes d'oxygène.

10. Procédé de préparation d'une molécule biologique selon l'une quelconque des revendications 6 à 9 où T est un acide aminé, **caractérisé en ce qu'**il comprend une étape de transformation du composé de formule générale (I), où Z comprend un acide carboxylique terminal, en ester d'hydroxysuccinimide et une étape de réaction avec T.

11. Procédé de préparation d'une molécule biologique selon la revendication 6 ou la revendication 7 où T est la biotine ou un de ses dérivés ou analogues structuraux, **caractérisé en ce qu'**il est mis en oeuvre à partir d'un composé de formule générale (I) fonctionnalisé avec un groupement Z porteur d'un acide carboxylique ; ledit composé (I) est (i) transformé en amide par une réaction d'hydrolyse avec une diamine aliphatique puis (ii) mis à réagir avec une biotine ou un de ses dérivés ou analogues structuraux possédant une fonction acide carboxylique libre.

12. Procédé de préparation d'une molécule biologique selon la revendication 6 ou la revendication 7 où T est un nucléotide, **caractérisé en ce qu'**il est mis en oeuvre à partir d'un composé de formule générale (I) fonctionnalisé avec un groupement Z porteur d'un acide carboxylique; ledit composé (I) étant mis à réagir avec un nucléotide modifié avec une fonction amine libre.

13. Méthode de détection d'un ligand d'une molécule biologique marquée selon l'une quelconque des revendications 6 à 9 dans un échantillon comprenant les étapes suivantes :
a) mesure du spectre d'émission de fluorescence de l'échantillon à tester seul ; le spectre obtenu est désigné « ligne de base » ;
b) mesure du spectre d'émission de fluorescence de la molécule biologique marquée en solution ; soustraction de la ligne de base au spectre obtenu et calcul de l'indice de déformation ;
c) incubation de ladite molécule biologique marquée en solution avec l'échantillon à tester et obtention d'un mélange ;
d) mesure du spectre d'émission de fluorescence du mélange obtenu à l'étape c) ; soustraction de la ligne de base au spectre obtenu et calcul de l'indice de déformation ;
e) comparaison des indices de déformation calculés aux étapes b) et d) et détection de l'interaction entre le ligand et la molécule biologique marquée lorsque lesdits indices sont différents.

14. Méthode de diagnostic de la maladie d'Alzheimer, **caractérisée** en qu'elle met en oeuvre la méthode selon la revendication 13 dans laquelle le ligand est le peptide bêta-amyloïde 1-42; l'échantillon contient des érythrocytes et la molécule biologique marquée est selon la revendication 9.

15. Méthode d'identification d'un composé capable d'interagir avec le ligand d'une molécule biologique marquée selon l'une quelconque des revendications 6 à 9 comprenant les étapes suivantes :
a) mesure du spectre d'émission de fluorescence et calcul de l'indice de déformation de la molécule biologique marquée en solution ;
b) incubation de ladite molécule biologique marquée en solution avec un ligand et obtention d'un mélange 1 ;
c) mesure du spectre d'émission de fluorescence et calcul de l'indice de déformation du mélange 1 ;
d) incubation dudit ligand avec ledit composé à tester puis ajout de ladite molécule biologique marquée en solution et obtention d'un mélange 2 ;
e) mesure du spectre d'émission de fluorescence et calcul de l'indice de déformation du mélange 2 ;
f) détection de l'interaction entre le ligand et le composé à tester par comparaison des indices de déformation calculés aux étapes a), c) et e).

16. Trousse de diagnostic pour la mise en oeuvre de la méthode selon la revendication 13 ou 14, **caractérisée en ce qu'**elle comprend au moins une molécule biologique marquée selon l'une quelconque des revendications 6 à 9.

## Patentansprüche

1. Verfahren zur Herstellung einer Verbindung der allgemeinen Formel (I): 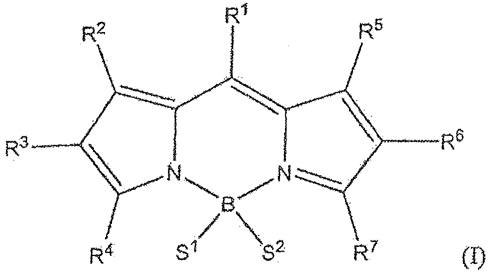 wobei:
- **R¹** aus der Gruppe ausgewählt ist, die von -Ar-CO-Z; einem Wasserstoffatom (-H); -L-H; -G und -L-G gebildet ist;
wobei Ar, Z, L und G nachfolgend definiert sind;
- **R³, R⁴, R⁶** und **R⁷** unabhängig voneinander aus der Gruppe ausgewählt sind, die von -(Ar)ₘ-CO-Z; einem Wasserstoffatom (-H); -L-H; -G und -L-G gebildet ist;
wobei m 0 oder 1 ist;
wobei es sich versteht, dass nur einer der Substituenten R¹, R³, R⁴, R⁶ und R⁷ -Ar-CO-Z oder -(Ar)ₘ-CO-Z ist;
- **R**² und **R**⁵, identisch oder unterschiedlich, unabhängig voneinander aus der Gruppe ausgewählt sind, die von einem Wasserstoffatom (-H); -L-H; -G und -L-G gebildet ist;
wobei L und G nachfolgend definiert sind;
- **S¹** und **S²** hydrophile Gruppen, identisch oder unterschiedlich, der Formel -C≡C-L'-A sind;
wobei L' und A nachfolgend definiert sind;
- **Ar** aus einem Arylen oder einem Heteroarylen mit C₅-C₁₄ ausgewählt ist, bei dem die Gruppe -CO-Z in Ortho-, Meta- oder Para-, vorzugsweise Paraposition ist;
- **Z** aus einer Gruppe -OH, -O-Succinimid, -O-Maleimid, -N-Glycin, -N-Lysin, -Y-L"-NH₂, -Y-L"-COOH, -Y-L"-SH ausgewählt ist, wobei **Y** aus den Atomen von N und O ausgewählt ist und **L"** nachfolgend definiert ist;
- **L** und **L"** unabhängig voneinander aus einer Einfachbindung; einer gesättigten kohlenstoffhaltigen Kette, eventuell verzweigt mit C₁-C₁₀, vorzugsweise mit C₁-C₆; einem Arylen mit C₆-C₁₆, auf dem die Gruppen -H oder -G für L und - NH₂, -COOH oder -SH für L" in Ortho-, Meta- oder Para-, vorzugsweise Paraposition sind; einem Alkenylen mit C₂-C₄; einem Alkynylen mit C₂-C₄; einer linearen oder verzweigten kohlenstoffhaltigen Kette mit C₁-C₂₀, unterbrochen von 1 bis 10 Sauerstoffatomen; einer linearen oder verzweigten kohlenstoffhaltigen Kette mit C₁-C₂₀, unterbrochen von einer bis vier Amidfunktionen -CO-NH-; einem Nukleotidsegment und/oder einem Zuckersegment ausgewählt sind;
- **G** aus der Gruppe ausgewählt ist, die von dem Succinimidylester, dem Sulfosuccinimidylester, dem Isothiocyanat, dem Isocyanat, dem Jodacetamid, dem Maleimid, den Halosulfonylen, den Phosphoramiditen, den Alkylimidaten mit
C₂-C₅, den Arylimidaten mit C₆-C₁₀, den Halogensäuren, vorzugsweise, den Chlorsäuren und den Bromsäuren, den Hydrazinen, substituiert durch ein Alkyl mit C₁-C₄, den Hydroxylaminen, substituiert durch ein Alkyl mit C₁-C₄, den Carbodiimiden und den Perfluorphenolen gebildet ist;
- **L'** aus einer Einfachbindung; einem Alkenylen mit C₁-C₁₀ oder einer gesättigten kohlenstoffhaltigen Kette, linear oder verzweigt mit C₁-C₂₀, unterbrochen von 1 bis 10 Sauerstoffatomen, ausgewählt ist;
- **A** eine Gruppe darstellt, die aus einem Alkyl mit C₁-C₄, vorzugsweise, dem Methyl; einer Phosphatgruppe oder einer Sulfonatgruppe ausgewählt ist,
wobei das Verfahren **dadurch gekennzeichnet ist, dass** es die Transformation des Synthesezwischenprodukts **P-(Ar)ₘ-Q** in Verbindung der allgemeinen Formel (I) durch Reaktion mit einer Carbonquelle bei Anwesenheit eines Nukleophils, ausgewählt aus dem Wasser, einem Alkohol, einem Amin oder einem Thiol und eines Palladiumkatalysators, umfasst;
wobei das Zwischenprodukt derart ist, dass P eine Strukturgruppe ist, die mit der herzustellenden Verbindung der allgemeinen Formel (I) identisch ist, mit Ausnahme des Radikals R¹ oder R³ oder R⁴ oder R⁶ oder R⁷, je nachdem, was die Gruppe -Ar-CO-Z für R¹ oder -(Ar)ₘ-CO-Z für R³ oder R⁴ oder R⁶ oder R⁷ ist, wobei das Radikal eine an -(Ar)ₘ-Q fixierte Bindung ist; wobei **Ar** so wie für die Formel (I) definiert ist, **m** 0 oder 1 ist, wobei es sich versteht, dass m 1 ist, wenn R¹ die Carbonylfunktion -Ar-CO-Z ist und **Q** aus einem Halogenatom; einem -O-Triflat; einem -O-Tosylat oder einem -O-Mesylat ausgewählt ist.

2. Verfahren nach Anspruch 1, **dadurch gekennzeichnet, dass** R² und R⁵ identisch sind, R³ und R⁶ identisch sind, R⁴ und R⁷ identisch sind und S¹ und S² identisch sind.

3. Verfahren nach einem der vorangehenden Ansprüche, **dadurch gekennzeichnet, dass** Ar aus dem Benzol, dem Naphtalen, dem Anthracen, dem Pyren, dem Pyridin, dem Pyrimidin, dem Thiophen oder dem Pyrrol ausgewählt ist.

4. Verfahren nach einem der vorangehenden Ansprüche, **dadurch gekennzeichnet, dass** A aus einem Methyl, einem Propylsulfonat, einem Ethylsulfonat oder einem Methylphosphat ausgewählt ist.

5. Verfahren nach einem der vorangehenden Ansprüche, **dadurch gekennzeichnet, dass** die gesättigte kohlenstoffhaltige Kette, linear oder verzweigt mit C₁-C₂₀, unterbrochen von 1 bis 10 Sauerstoffatomen, ein (Poly)Ethylenoxid oder ein (Poly)Propylenoxid ist, dessen Motiv sich zwischen einem und sechs Mal wiederholt.

6. Markiertes biologisches Molekül der allgemeinen Formel (II):
P-(Ar)ₘ-CO-(X)ₙ-T (II)
wobei:
- **P** die Verbindung der allgemeinen Formel (I) ist, wobei:
- R¹ aus der Gruppe ausgewählt ist, die von -Ar-CO-Z; einem Wasserstoffatom (-H); -L-H; -G und -L-G gebildet ist;
wobei Ar, Z, L und G nachfolgend definiert sind;
- R³, R⁴, R⁶ und R⁷ unabhängig voneinander aus der Gruppe ausgewählt sind, die von -(Ar)ₘ-CO-Z; einem Wasserstoffatom (-H); -L-H; -G und -L-G gebildet ist;
wobei m 0 oder 1 ist;
wobei es sich versteht, dass nur einer der Substituenten R¹, R³, R⁴, R⁶ und R⁷ -Ar-CO-Z oder -(Ar)ₘ-CO-Z ist;
- R² und R⁵, identisch oder unterschiedlich, unabhängig voneinander aus der Gruppe ausgewählt sind, die von einem Wasserstoffatom (-H); -L-H; -G und -L-G gebildet ist;
wobei L und G nachfolgend definiert sind;
- S¹ und S² hydrophile Gruppen, identisch oder unterschiedlich, der Formel -C≡C-L'-A sind;
wobei L' und A nachfolgend definiert sind;
- Ar aus einem Arylen oder einem Heteroarylen mit C₅-C₁₄ ausgewählt ist, bei dem die Gruppe -CO-Z in Ortho-, Meta- oder Para-, vorzugsweise Paraposition ist;
- Z aus einer Gruppe -OH, -O-Succinimid, -O-Maleimid, -N-Glycin, -N-Lysin, -Y-L"-NH₂, -Y-L"-COOH, -Y-L"-SH ausgewählt ist, wobei Y aus den Atomen von N und O ausgewählt ist und L" nachfolgend definiert ist;
- L und L" unabhängig voneinander aus einer Einfachbindung; einer gesättigten kohlenstoffhaltigen Kette, eventuell verzweigt mit C₁-C₁₀, vorzugsweise mit C₁-C₆; einem Arylen mit C₆-C₁₆, auf dem die Gruppen -H oder -G für L und - NH₂, -COOH oder -SH für L" in Ortho-, Meta- oder Para-, vorzugsweise Paraposition sind; einem Alkenylen mit C₂-C₄; einem Alkynylen mit C₂-C₄; einer linearen oder verzweigten kohlenstoffhaltigen Kette mit C₁-C₂₀, unterbrochen von 1 bis 10 Sauerstoffatomen; einer linearen oder verzweigten kohlenstoffhaltigen Kette mit C₁-C₂₀, unterbrochen von einer bis vier Amidfunktionen -CO-NH-; einem Nukleotidsegment und/oder einem Zuckersegment ausgewählt sind;
- G aus der Gruppe ausgewählt ist, die von dem Succinimidylester, dem Sulfosuccinimidylester, dem Isothiocyanat, dem Isocyanat, dem Jodacetamid, dem Maleimid, den Halosulfonylen, den Phosphoramiditen, den Alkylimidaten mit
C₂-C₅, den Arylimidaten mit C₆-C₁₀, den Halogensäuren, vorzugsweise, den Chlorsäuren und den Bromsäuren, den Hydrazinen, substituiert durch ein Alkyl mit C₁-C₄, den Hydroxylaminen, substituiert durch ein Alkyl mit C₁-C₄, den Carbodiimiden und den Perfluorphenolen gebildet ist;
- L' aus einer Einfachbindung; einem Alkenylen mit C₁-C₁₀ oder einer gesättigten kohlenstoffhaltigen Kette, linear oder verzweigt mit C₁-C₂₀, unterbrochen von 1 bis 10 Sauerstoffatomen, ausgewählt ist;
- A eine Gruppe darstellt, die aus einem Alkyl mit C₁-C₄, vorzugsweise, dem Methyl; einer Phosphatgruppe oder einer Sulfonatgruppe ausgewählt ist,
mit Ausnahme des Radikals R¹ oder R³ oder R⁴ oder R⁶ oder R⁷, das die Gruppe -(Ar)ₘ-CO-Z in der allgemeinen Formal (I) ist, wobei das Radikal dann eine Bindung an -(Ar)ₘ-CO-(X)ₙ-T ist;
- **Ar** aus einem Arylen oder einem Heteroarylen mit C₅-C₁₀ ausgewählt ist, bei dem die Gruppe -CO-Z in Ortho-, Meta- oder Para-, vorzugsweise Paraposition ist;
- **m** 0 oder 1 ist, wobei es sich versteht, dass m 1 ist, wenn die Gruppe -(Ar)ₘ-CO-(X)ₙ-T gegen R¹ substituiert ist,
- **X** ein Spacer ist, Träger eine Carboxyl-, Amin- oder Thiolfunktion;
- **n** eine Ganzzahl gleich 0 oder 1 ist;
- **T** ein biologisches Molekül ist.

7. Markiertes biologisches Molekül nach Anspruch 6, **dadurch gekennzeichnet, dass** T aus einer natürlichen oder synthetischen Aminosäure, einem Polypeptid, Biotin oder einem seiner Derivate oder strukturell Ähnlichem, einem Nukleotid oder einer Nukleinsäure ausgewählt ist.

8. Markiertes biologisches Molekül nach Anspruch 6 oder 7, **dadurch gekennzeichnet, dass** T ein vom Peptid Beta-Amyloid 1-42 SEQ ID NO: 1 abgeleitetes Peptid ist.

9. Markiertes biologisches Molekül nach einem der Ansprüche 6 bis 8, **dadurch gekennzeichnet, dass** X aus einer Kette ausgewählt ist, die mindestens eine Aminosäure oder ein Alkylen mit C₁-C₆ umfasst, möglicherweise unterbrochen von 2 oder 3 Sauerstoffatomen.

10. Verfahren zur Herstellung eines biologischen Moleküls nach einem der Ansprüche 6 bis 9, wobei T eine Aminosäure ist, **dadurch gekennzeichnet, dass** es einen Transformationsschritt der Verbindung der allgemeinen Formel (I) umfasst, wobei Z eine terminale Carbonsäure, als Hydroxysuccinimidester und einen Reaktionsschritt mit T umfasst.

11. Verfahren zur Herstellung eines biologischen Moleküls nach Anspruch 6 oder Anspruch 7, wobei T Biotin oder eines seiner Derivate oder strukturell Ähnliches ist, **dadurch gekennzeichnet, dass** es auf der Basis einer Verbindung der allgemeinen Formel (I), funktionalisiert mit einer Gruppe Z als Träger einer Carbonsäure, umgesetzt wird; wobei die Verbindung (I) (i) durch eine Hydrolysereaktion mit einem aliphatischen Diamin in Amid transformiert wird, dann (ii) in Reaktion mit einem Biotin oder einem seiner Derivate oder strukturell Ähnlichem mit einer freien Carbonsäurefunktion versetzt wird.

12. Verfahren zur Herstellung eines biologischen Moleküls nach Anspruch 6 oder Anspruch 7, wobei T ein Nukleotid ist, **dadurch gekennzeichnet, dass** es auf der Basis einer Verbindung der allgemeinen Formel (I), funktionalisiert mit einer Gruppe Z als Träger einer Carbonsäure, umgesetzt wird; wobei die Verbindung (I) mit einem mit einer freien Aminfunktion modifiziertem Nukleotid in Reaktion versetzt wird.

13. Methode zur Detektion eines Liganden eines markierten biologischen Moleküls nach einem der Ansprüche 6 bis 9 in einer Probe, umfassend die folgenden Schritte:
a) Messen des Fluoreszenzemissionsspektrums nur der zu testenden Probe, wobei das erhaltene Spektrum als "Basislinie" bezeichnet wird;
b) Messen des Fluoreszenzemissionsspektrums des markierten biologischen Moleküls in Lösung; Subtraktion der Basislinie von dem erhaltenen Spektrum und Berechnen des Verformungsindexes;
c) Inkubieren des markierten biologischen Moleküls in Lösung mit der zu testenden Probe und Erhalten eines Gemischs;
d) Messen des Fluoreszenzemissionsspektrums des in Schritt c) erhaltenen Gemischs; Subtraktion der Basislinie von dem erhaltenen Spektrum und Berechnen des Verformungsindexes;
e) Vergleichen der in den Schritten b) und d) berechneten Verformungsindexe und Detektion der Interaktion zwischen dem Liganden und dem markierten biologischen Molekül, wenn die Indexe unterschiedlich sind.

14. Methode zur Diagnose der Alzheimerkrankheit, **dadurch gekennzeichnet, dass** sie die Methode nach Anspruch 13 umsetzt, wobei der Ligand das Peptid Beta-Amyloid 1-42 ist; wobei die Probe Erythrocyten enthält und das markierte biologische Molekül nach Anspruch 9 ist.

15. Method zur Identifizierung einer Verbindung, die imstande ist, mit dem Liganden eines markierten biologischen Moleküls nach einem der Ansprüche 6 bis 9 zu interagieren, umfassend die folgenden Schritte:
a) Messen des Fluoreszenzemissionsspektrums und Berechnen des Verformungsindexes des markierten biologischen Moleküls in Lösung;
b) Inkubieren des markierten biologischen Moleküls in Lösung mit einem Liganden und Erhalten eines Gemischs 1;
c) Messen des Fluoreszenzemissionsspektrums und Berechnen des Verformungsindexes des Gemischs 1;
d) Inkubieren des Liganden mit der zu testenden Verbindung, danach Hinzufügen des markierten biologischen Moleküls in Lösung und Erhalten eines Gemischs 2;
e) Messen des Fluoreszenzemissionsspektrums und Berechnen des Verformungsindexes des Gemischs 2;
f) Ermitteln der Interaktion zwischen dem Liganden und der zu testenden Verbindung durch Vergleichen der in den Schritten a), c) und e) berechneten Verformungsindexe.

16. Diagnosekit für die Umsetzung der Methode nach Anspruch 13 oder 14, **dadurch gekennzeichnet, dass** es mindestens ein markiertes biologisches Molekül nach einem der Ansprüche 6 bis 9 umfasst.

## Claims

1. Method for preparing a compound of general formula (I): 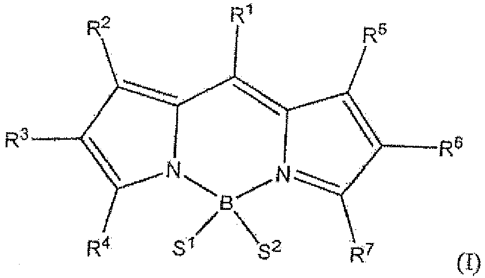 where:
- **R¹** is selected from the group formed by -Ar-CO-Z; a hydrogen atom (-H); - L-H; -G and -L-G;
with Ar, Z, L and G being defined below;
- **R³, R⁴, R⁶** and **R⁷** are each independently selected from the group formed by -(Ar)ₘ-CO-Z; a hydrogen atom (-H); -L-H; -G and -L-G;
where m is 0 or 1;
on the understanding that only one of the substituents R¹, R³, R⁴, R⁶ and R⁷ is -Ar-CO-Z or -(Ar)ₘ-CO-Z;
- **R²** and R**⁵,** the same or different, are each independently selected from the group formed by a hydrogen atom (-H); -L-H; -G and -L-G;
with L and G being defined below;
- **S¹** and **S²** are hydrophilic groups, the same or different, of formula -C≡C-L'-A;
with L' and A being defined below;
- **Ar** is selected from among a C₅-C₁₄ arylene or heteroarylene on which the group -CO-Z is at ortho, meta or para position preferably para;
- **Z** is selected from among the groups -OH, -O-succinimide, -O-maleimide, - N-glycine, -N-lysine, -Y-L"-NH₂, -Y-L"-COOH, -Y-L"-SH where **Y** is selected from among the atoms of N and O and **L"** is defined below;
- **L** and **L"** are each independently selected from among a single bond; a saturated, optionally branched C₁-C₁₀ carbon chain, preferably C₁-C₆; a C₆-C₁₆ arylene on which the groups -H or -G for L and -NH₂, -COOH or -SH for L" are at ortho, meta or para position preferably para; a C₂-C₄ alkenylene; C₂-C₄ alkynylene; straight-chain or branched C₁-C₂₀ carbon chain interrupted by 1 to 10 oxygen atoms; a saturated, straight-chain or branched C₁-C₂₀ carbon chain interrupted by one to four -CO-NH- amide functions, a nucleotide segment and/or sugar segment;
- **G** is selected from the group formed by the succinimidyl ester, sulfosuccinimidyl ester, isothiocyanate, isocyanate, iodoacetamide, maleimide, halosulfonyls, phosphoramidites, C₂-C₅ alkylimidates, C₆-C₁₀ arylimidates, halogeno-acids preferably chloroacids and bromoacids, hydrazines substituted by a C₁-C₄ alkyl, hydroxylamines substituted by a C₁-C₄ alkyl, carbodiimides and perfluorophenols;
- **L'** is selected from among a single bond; a C₁-C₁₀ alkenylene or a saturated, straight-chain or branched C₁-C₂₀ carbon chain interrupted by 1 to 10 oxygen atoms;
- **A** is a group selected from among a C₁-C₄ alkyl, preferably methyl; a phosphate group or sulfonate group,
said method being **characterized in that** it comprises the conversion of the synthesis intermediate **P-(Ar)ₘ-Q** to a compound of general formula (I) by reaction with a carbon source in the presence of a nucleophile selected from among water, an alcohol, an amine or thiol and a palladium catalyst;
said intermediate is such that **P** is a group having identical structure with the compound of general formula (I) to be prepared with the exception of the radical R¹ or R³ or R⁴ or R⁶ or R⁷ according to which one is the -Ar-CO-Z group for R¹ or -(Ar)ₘ-CO-Z group for R³ or R⁴ or R⁶ or R⁷, said radical being a bond attached to -(Ar)ₘ-Q; **Ar** is such as defined for formula (I), **m** is 0 or 1, on the understanding that m is 1 if R¹ is the carbonyl function -Ar-CO-Z, and **Q** is selected from among a halogen atom; an -O-triflate; O-tosylate or -O-mesylate.

2. The method according to claim 1, **characterized in that** R² and R⁵ are the same, R³ and R⁶ are the same, R⁴ and R⁷ are the same and S¹ and S² are the same.

3. The method according to any of the preceding claims, **characterized in that** Ar is selected from among benzene, naphthalene, anthracene, pyrene, pyridine, pyrimidine, thiophene or pyrrole.

4. The method according to any of the preceding claims **characterized in that** A is selected from among a methyl, propyl sulfonate, ethyl sulfonate or methylphosphate.

5. The method according to any of the preceding claims, **characterized in that** the saturated, straight-chain or branched C₁-C₂₀ carbon chain interrupted by 1 to 10 oxygen atoms is a poly(ethylene oxide) or polypropylene oxide) having a repeat unit repeating between one and six times.

6. A labelled biological molecule of general formula (II):
P-(Ar)ₘ-CO-(X)ₙ-T (II)
where:
- **P** is the compound of general formula (I)
where:
- **R¹** is selected from the group formed by -Ar-CO-Z; a hydrogen atom (-H); - L-H; -G and -L-G;
with Ar, Z, L and G being defined below;
- **R³, R⁴, R⁶** and **R⁷** are each independently selected from the group formed by -(Ar)ₘ-CO-Z; a hydrogen atom (-H); -L-H; -G and -L-G;
where m is 0 or 1;
on the understanding that only one of the substituents R¹, R³, R⁴, R⁶ and R⁷ is -Ar-CO-Z or -(Ar)ₘ-CO-Z;
- **R²** and **R⁵,** the same or different, are each independently selected from the group formed by a hydrogen atom (-H); -L-H; -G and -L-G;
with L and G being defined below;
- **S¹** and **S²** are hydrophilic groups, the same or different, of formula -C≡C-L'-A;
with L' and A being defined below;
- **Ar** is selected from among a C₅-C₁₄ arylene or heteroarylene on which the group -CO-Z is at ortho, meta or para position preferably para;
- **Z** is selected from among the groups -OH, -O-succinimide, -O-maleimide, - N-glycine, -N-lysine, -Y-L"-NH₂, -Y-L"-COOH, -Y-L"-SH where Y is selected from among the atoms of N and O and L" is defined below;
- **L** and **L"** are each independently selected from among a simple bond; a saturated, optionally branched C₁-C₁₀ carbon chain, preferably C₁-C₆; a C₆-C₁₆ arylene on which the groups -H or -G for L and -NH₂, -COOH or -SH for L" are at ortho, meta or para position preferably para; a C₂-C₄ alkenylene; C₂-C₄ alkynylene; straight-chain or branched C₁-C₂₀ carbon chain interrupted by 1 to 10 oxygen atoms; a saturated, straight-chain or branched C₁-C₂₀ carbon chain interrupted by one to four -CO-NH- amide functions, a nucleotide segment and/or sugar segment;
- **G** is selected from the group formed by the succinimidyl ester, sulfosuccinimidyl ester, isothiocyanate, isocyanate, iodoacetamide, maleimide, halosulfonyls, phosphoramidites, C₂-C₅ alkylimidates, C₆-C₁₀ arylimidates, halogeno-acids preferably chloroacids and bromoacids, hydrazines substituted by a C₁-C₄ alkyl, hydroxylamines substituted by a C₁-C₄ alkyl, carbodiimides and perfluorophenols;
- **L'** is selected from among a single bond; a C₁-C₁₀ alkenylene or a saturated, straight-chain or branched C₁-C₂₀ carbon chain interrupted by 1 to 10 oxygen atoms;
- **A** is a group selected from among a C₁-C₄ alkyl, preferably methyl; a phosphate group or sulfonate group,
with the exception of the radical R¹ or R³ or R⁴ or R⁶ or R⁷ which is the group-(Ar)ₘ-CO-Z in general formula (I), said radical is then a bond with - (Ar)ₘ-CO-(X)ₙ-T;
- **Ar** is selected from among a C₅-C₁₀ arylene or heteroarylene on which the-CO-Z group is at ortho, meta or para position, preferably para;
- **m** is 0 or 1 on the understanding that m is 1 if the group -(Ar)ₘ-CO-(X)ₙ-T is substituted for R¹;
- **X** is a spacer carrying a carboxylic, amine or thiol function;
- **n** is an integer of 0 or 1;
- **T** is a biological molecule.

7. The labelled biological molecule according to claim 6, **characterized in that** T is selected from among a natural or synthetic amino acid, a polypeptide, biotin or one of the structural derivatives or analogues thereof, a nucleotide or nucleic acid.

8. The labelled biological molecule according to claim 6 or 7, **characterized in that** T is a peptide derived from the beta-amyloid (1-42) peptide of SEQ ID NO:1.

9. The labelled biological molecule according to any of claims 6 to 8, **characterized in that** X is selected from among a chain comprising at least one amino acid or a C₁-C₆ alkylene possibly interrupted by 2 or 3 oxygen atoms.

10. Method for preparing a biological molecule according to any of claims 6 to 9 where T is an amino acid, **characterized in that** it comprises a step to convert the compound of general formula (1), where Z comprises a terminal carboxylic acid, to a hydroxysuccinimide ester, and a reaction step with T.

11. The method for preparing a biological molecule according to claim 6 or claim 7 where T is biotin or one of the structural derivatives or analogues thereof, **characterized in that** it is implemented starting with a compound of general formula (I) functionalized with a Z group carrying a carboxylic acid; said compound (I) is (i) converted to an amide by hydrolysis reaction with an aliphatic diamine, then (ii) reacted with a biotin or one of the structural derivatives or analogues thereof having a free carboxylic acid function.

12. The method for preparing a biological molecule according to claim 6 or claim 7 where T is a nucleotide, **characterized in that** it is implemented starting with a compound of general formula (I) functionalized with a Z group carrying a carboxylic acid; said compound (I) is reacted with a nucleotide modified with a free amine function.

13. Method for detecting a ligand of a labelled biological molecule according to any of claims 6 to 9, in a sample, comprising the following steps:
a) measuring the fluorescence emission spectrum of the sample to be tested alone; the spectrum obtained is designated the "baseline";
b) measuring the fluorescence emission spectrum of the labelled biological molecule in solution; subtracting the baseline from the spectrum obtained and calculating the deformation index;
c) incubating said labelled biological molecule in solution with the sample to be tested, to obtain a mixture;
d) measuring the fluorescence emission spectrum of the mixture obtained at step c); subtracting the baseline from the spectrum obtained and calculating the deformation index;
e) comparing the deformation indexes calculated at steps b) and d) and detecting the interaction between the ligand and the labelled biological molecule when said indexes are different.

14. Method for diagnosing Alzheimer's disease, **characterized in that** it applies the method according to claim 13 and wherein the ligand is the beta-amyloid (1-42) peptide; the sample contains erythrocytes and the labelled biological molecule is according to claim 9.

15. Method for identifying a compound capable of interacting with the ligand of a labelled biological molecule according to any of claims 6 to 9, comprising the following steps:
a) measuring the fluorescence emission spectrum and calculating the deformation index of the labelled biological molecule in solution;
b) incubating said labelled biological molecule in solution with a ligand, to obtain a mixture 1;
c) measuring the fluorescence emission spectrum and calculating the deformation index of mixture 1;
d) incubating said ligand with said compound to be tested, then adding said labelled biological molecule in solution, to obtain a mixture 2;
e) measuring the fluorescence emission spectrum and calculating the deformation index of mixture 2;
f) detecting the interaction between the ligand and the compound to be tested by comparing the deformation indexes calculated at steps a), c) and e).

16. Diagnosis kit to implement the method according to claim 13 or 14, **characterized in that** it comprises at least one labelled biological molecule according to any of claims 6 to 9.
